# EUROPEAN PATENT APPLICATION

(11) **EP 2 818 166 A1**
(43) Date of publication of application: **31.12.2014**
(21) Application number: 13382246.0
(22) Date of filing: 26.06.2013
(51) Int. Cl.: A61K 31/415, A61K 31/4155, A61K 31/485, A61K 31/5377, A61P 13/10, A61P 29/00

(54) **Use of sigma receptor ligands for the prevention and treatment of pain associated to interstitial cystitis/bladder pain syndrome (IC/BPS)**

(71) Applicant: Laboratorios del Dr. Esteve S.A., 08041 Barcelona (ES)
(72) Inventor: Vela Hernández, José-Miguel, 08028 Barcelona (ES); Merlos-Roca, Manuel, 08021 Barcelona (ES); Baeyens-Cabrera, José-Manuel, 18008 Granada (ES); Cendán-Martínez, Cruz-Miguel, 18014 Granada (ES)
(74) Representative: ABG Patentes, S.L.

(57) **Abstract**

The invention refers to the use of a sigma ligand, particularly a sigma ligand of formula (I) to prevent and/or treat pain associated to interstitial cystitis/bladder pain syndrome (IC/BPS).

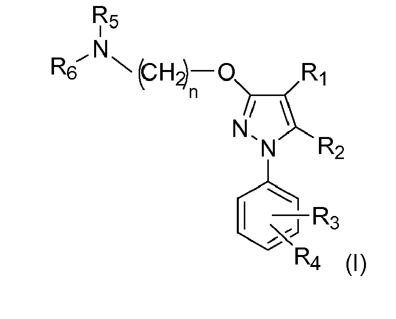

## Description

### FIELD OF THE INVENTION

The present invention relates to the use of Sigma receptor ligands, and more particularly to some pyrazole derivatives, as well as pharmaceutical compositions comprising them, in therapy and/or prophylaxis of pain associated to interstitial cystitis/bladder pain syndrome (IC/BPS).

### BACKGROUND

The treatment of pain conditions is of great importance in medicine. There is currently a worldwide need for additional pain therapies. The pressing requirement for a specific treatment of each pain condition is documented in the large number of scientific works that have appeared recently in the field of applied analgesics.

PAIN is defined by the International Association for the Study of Pain (IASP) as "an unpleasant sensory and emotional experience associated with actual or potential tissue damage, or described in terms of such damage" (IASP, Classification of chronic pain, 2nd Edition, IASP Press (2002), 210). Although it is a complex process influenced by both physiological and psychological factors and is always subjective, its causes or syndromes can be classified. Pain can be classified based on temporal, aetiological or physiological criteria. When pain is classified by time, it can be acute or chronic. Aetiological classifications of pain are malignant or non-malignant. A third classification is physiological, which includes nociceptive pain (results from detection by specialized transducers in tissues attached to A-delta and C-fibres), that can be divided into somatic and visceral types of pain, and neuropathic pain (results from irritation or damage to the nervous system), that can be divided into peripheral and central neuropathic pain. Pain is a normal physiological reaction of the somatosensory system to noxious stimulation which alerts the individual to actual or potential tissue damage. It serves a protective function of informing us of injury or disease, and usually remits when healing is complete or the condition is cured. However, pain may result from a pathological state characterized by one or more of the following: pain in the absence of a noxious stimulus (spontaneous pain), increased duration of response to brief stimulation (ongoing pain or hyperpathia), reduced pain threshold (allodynia), increased responsiveness to suprathreshold stimulation (hyperalgesia), spread of pain and hyperalgesia to uninjured tissue (referred pain and secondary hyperalgesia), and abnormal sensations (e.g., dysesthesia, paresthesia).

Cystitis or inflammation of the bladder has a direct effect on bladder function. It can occur due to both infectious (such as Gram-negative microorganisms, Gram-positive microorganisms or Group B streptococci) as well as non-infectious (medication, radiation, chemicals etc.) etiologies. It may even be idiopathic in nature such as interstitial cystitis (IC) or occur in association with other diseases. Irrespective of the cause, cystitis can be acute or chronic depending upon the duration of the insult.

The first and early response to any noxious stimulus or injury occurs in the form of inflammation which causes the release of the named mediators (such as cytokines, histamines, kinins etc.). In case of acute cystitis these mediators cause erythematous swelling and ulceration of the bladder mucosa, which bleeds easily. In general, these mediators have a short half-life and are quickly degraded, therefore enabling rapid resolution of inflammation as soon as the noxious stimulus is removed. However, if the stimulus is not removed, chronic inflammation ensues, such as seen in IC. This is characterized, among others, by hyperalgesia responsible for the chronic waxing and waning symptoms of pain and lower urinary tract symptoms (Sonal, G. et al.; Ther. Adv. Urol.; 2011; 3(1); 19-33).

Interstitial cystitis (IC) is a syndrome characterized by urinary bladder pain and irritative symptoms of more than 6 months duration. The constellation of IC symptoms has been given different names. The International Continence Society named the disease interstitial cystitis/painful bladder syndrome (IC/PBS) in 2002 (Abrahams et al.; Neurol. Urodyn.; 2002; 21; 167-178), while the Multinational Interstitial Cystitis Association have labelled it as painful bladder syndrome/interstitial cystitis (PBS/IC) (Hanno et al.; Int. Urogynecol. J. Pelvic Floor Dysfunct.; 2005; 16 (suppl. 1); S2-S34). Recently the European Society for the study of Interstitial Cystitis (ESSIC) proposed the moniker "bladder pain syndrome" (BPS) defining IC as chronic pelvic pain, pressure or discomfort perceived to be related to the urinary bladder and accompanied by at least one other urinary symptom such as persistent urge to void or urinary frequency (van de Merwe et al.; Eur. Urol.; 2005; 53; 60-67).

Many theories have been suggested to exemplify the pathogenesis behind IC. However a central role of inflammation has been confirmed in both human and animal studies using electron microscopy and immunohistochemical staining techniques. Irrespective of the ethiology, if the noxious stimulus persists for a longer duration, it leads chronic inflammation. As a result, a cascade of events, which are interrelated with each other is initiated, resulting in a vicious, self-reinforcing cycle of persistent inflammation and recurrent injury to bladder epithelium (Sant, G.R. et al.; Urology; 2007; 69 (4 suppl.); 34-40). Additionally, studies have suggested that the urothelium releases a number of substances which activates afferent nerves and mast cells during this process resulting in hyperalgesia in patients with IC (Theoharides, T.C. et al.; Urology; 2001; 57(6 Suppl.); 47-55).

It is believed that IC also represents a visceral neuropathic pain syndrome mediated by upregulation of nerves in the pelvis, spinal cord and brain. An increased sensitivity of bladder sensory afferents may thus also be responsible for increased pain sensation or hyperalgesia (Dmitrieva,N. et al.; Neuroscience; 1997; 78; 449-459).

It is now widely accepted that the diagnosis of IC should be based on symptoms along with the exclusion of similar, but different, conditions such as pelvic pain, urinary tract infection (UTI), yeast infections, endometriosis, pelvic organ prolapse, gynaecological or urological malignancies, overactive bladder and chronic prostatitis.

One of the current approaches for treating pain associated to IC/BPS (Sonal, G. et al.; Ther. Adv. Urol.; 2011; 3(1); 19-33) stands on a multimodal treatment regimen with:
- *oral therapies* which includes, among others, bladder mucosal protectors (pentosan polysulphate (Elmiron)); anti-allergics as antihistaminics (H1 blockers (hydroxyzine hydrochloride) as well as H2 blockers (Cimetidine)); leukotriene-D4 receptor antagonist montelukast; pain modulators as trycyclic antidepressants (Amitriptyline) or anticonvulsants (gabapentin); hormone modulators (Leuprolide acetate); anti-inflammatory agents as anti-TNF; narcotics; pain relief agents (opioids, Tramadol); immunosuppressive agents (Prednisone, Triamcinolone); L-arginine; Oxybutynin or Tolterodine;
- *intravesical therapies* which includes, among others, pain modulators (Dimethylsulfoxide or Bacillus Calmette-Guerin (BCG)), bladder mucosal protectors (Hyaluronic acid), or Chondroitin sulphate;
- *surgical treatment;* or
- *complementary therapies* that utilize anti-inflammatory, neural, anesthesic and behavioural agents.

In summary, as pain associated to IC/BPS also represents a visceral neuropathic pain syndrome that could be characterized, among others, by hyperalgesia and no standard treatment is known, there is a need to provide a new form of treatment for IC/BPS associated pain and especially for neuropathic pain, allodynia, hyperalgesia and peripheral neuropathy, developed during and/or after IC/BPS.

### BRIEF DESCRIPTION OF THE INVENTION

The inventors of the present invention have found and demonstrated that Sigma ligands can be useful in the therapy of interstitial cystitis/bladder pain syndrome (IC/BPS) associated pain.

Therefore, one aspect of the present invention relates to a Sigma receptor ligand for use in the treatment and/or prevention of pain associated to IC/BPS.

This benefit of the invention is more evident when the Sigma ligand is specifically a Sigma-1 receptor antagonist, preferably in the form of a (neutral) antagonist, an inverse agonist or a partial antagonist.

In a preferred embodiment, said Sigma ligand has the general formula (I): 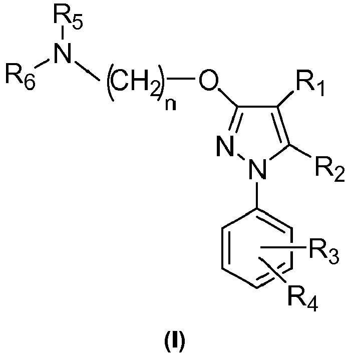 wherein
**R₁** is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted aromatic or non-aromatic heterocyclyl, substituted or unsubstituted heterocyclylalkyl, -COR₈, -C(O)OR₈, - C(O)NR₈R₉, -CH=NR₈, -CN, -OR₈, -OC(O)R₈, -S(O)ₜ-R₈, -NR₈R₉, -NR₈C(O)R₉, - NO₂, -N=CR₈R₉, and halogen;
**R₂** is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, -C(O)OR₈, - C(O)NR₈R₉, -CH=NR₈, -CN, -OR₈, -OC(O)R₈, -S(O)ₜ-R₈, -NR₈R₉, -NR₈C(O)R₉, - NO₂, -N=CR₈R₉, and halogen;
**R₃** and **R₄** are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, - COR₈, -C(O)OR₈, -C(O)NR₈R₉, -CH=NR₈, -CN, -OR₈, -OC(O)R₈, -S(O)ₜ-R₈, - NR₈R₉, -NR₈C(O)R₉, -NO₂, -N=CR₈R₉, and halogen; or together with the phenyl they form an optionally substituted fused ring system;
**R₅** and **R₆** are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, - COR₈, -C(O)OR₈, -C(O)NR₈R₉, -CH=NR₈, -CN, -OR₈, -OC(O)R₈, -S(O)ₜ-R₈, - NR₈R₉, -NR₈C(O)R₉, -NO₂, -N=CR₈R₉, and halogen;
or together form, with the nitrogen atom to which they are attached, a substituted or unsubstituted, aromatic or non-aromatic heterocyclyl group;
**n** is selected from 1, 2, 3, 4, 5, 6, 7 and 8;
**t is 0,** 1 or 2;
**R₈** and **R₉** are each independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted, aromatic or non-aromatic heterocyclyl, substituted or unsubstituted alkoxy, substituted or unsubstituted aryloxy, and halogen;
or a pharmaceutically acceptable salt, isomer, prodrug or solvate thereof.

Another aspect of this invention refers to the use of a Sigma receptor ligand, preferably a Sigma ligand of general formula (I), or a pharmaceutically acceptable salt, isomer, prodrug or solvate thereof, for the manufacture of a medicament for the treatment and/or prevention of pain associated to IC/BPS.

Another aspect of the invention is a method of treatment of a patient suffering, or likely to suffer, pain associated to IC/BPS, which comprises administering to the patient in need of such a treatment or prophylaxis a therapeutically effective amount of a Sigma receptor ligand, preferably a Sigma ligand of general formula (I), or a pharmaceutically acceptable salt, isomer, prodrug or solvate thereof.

Another aspect of the invention refers to a medicament or pharmaceutical composition comprising at least one Sigma receptor ligand and at least one pharmaceutically acceptable excipient for use in the treatment and/or prevention of pain associated to IC/BPS.

Another aspect of the invention refers to a combination of at least one Sigma receptor ligand and at least one further active substance for use in the treatment and/or prevention of pain associated to IC/BPS.

These aspects and preferred embodiments thereof are additionally also defined hereinafter in the detailed description, as well as in the claims.

### BRIEF DESCRIPTION OF THE FIGURES

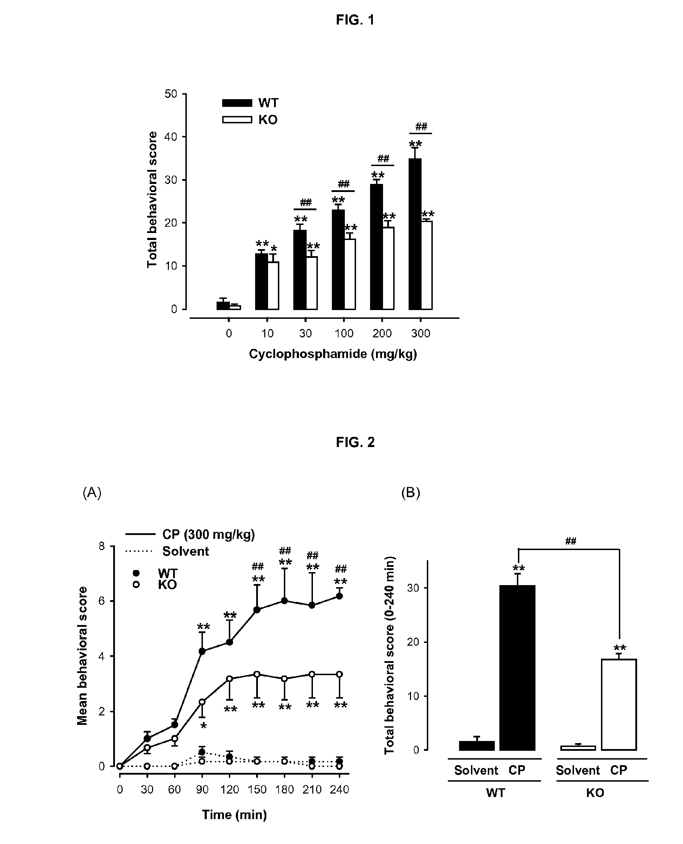

**Figure 1****:** Pain-related behaviors induced by i.p. administration of different doses of cyclophosphamide (10-300 mg/kg) or its solvent (0) in wild-type (WT) and σ₁ receptor knockout (KO) mice. The behavioral pain responses were recorded at 30 min intervals over the 4 h observation period after the cyclophosphamide or its solvent injection. Each bar and vertical line represents the mean ± SEM of values obtained in 10-12 animals. Statistically significant differences between the values obtained in cyclophosphamide- and solvent-treated animals: **p* < 0.05; ***p* < 0.01; and between the values obtained in wild-type and σ₁ knockout animals at the same dose of cyclophosphamide: ^{##}*p* < 0.01 (two-way ANOVA followed by Bonferroni test).
**Figure 2****:** Pain-related behaviors induced by i.p. administration of cyclophosphamide (CP 300 mg/kg) or its solvent in wild-type (WT) and σ₁ receptor knockout (KO) mice. (A) Time-course recording the behavioral pain at 30 min intervals over the 4 h observation period after the cyclophosphamide injection. (B) Total behavioral score representing the behavioral pain responses at 30 min intervals over the complete observation period (0-240 min). Each point or bar and vertical line represents the mean ± SEM of values obtained in 10-12 animals. Statistically significant differences between the values obtained in cyclophosphamide- and solvent-treated animals: **p* < 0.05; ***p* < 0.01; and between the values obtained in wild-type and σ₁ knockout animals at the same dose of cyclophosphamide: (A) ^{##} *p* < 0.01 (two-way ANOVA followed by Bonferroni test). (B) ^{##} *p* < 0.01 (Student t test).

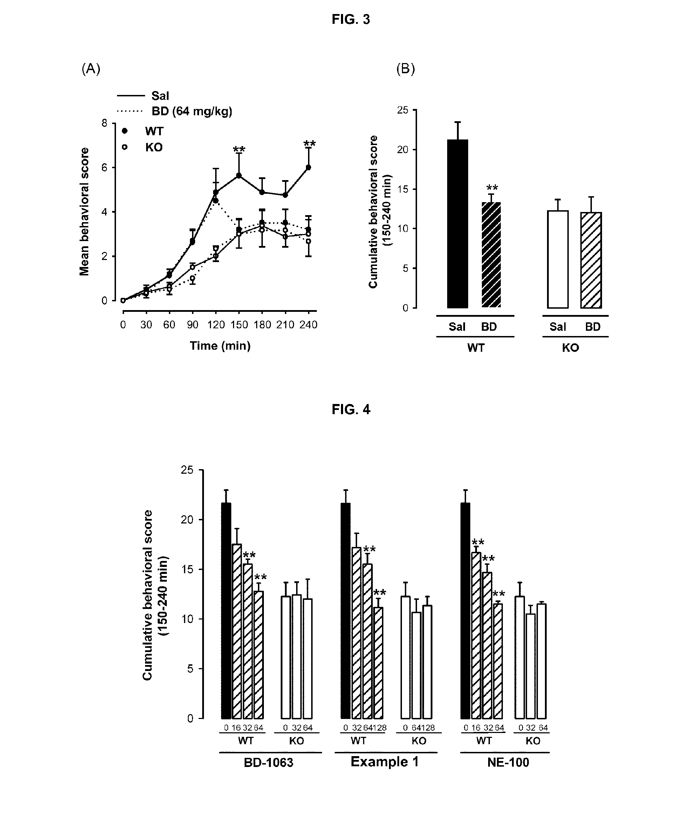

**Figure 3****:** Effects of the s.c. administration of BD-1063 (BD; 64 mg/kg) or saline (Sal) on the pain-related behaviors evoked by i.p. administration of cyclophosphamide (CP; 300 mg/kg) in wild-type (WT; •) and σ₁ receptor knockout (KO; ○) mice. (A) Time-course recording the behavioral pain responses at 30 min intervals over a 240 min observation period. (B) Cumulative behavioral score representing the behavioral pain responses at 30 min intervals over the 150-240 min observation period. BD-1063 or saline was injected at 120 min after the administration of cyclophosphamide or its solvent. Each point or bar and vertical line represents the mean ± SEM of values obtained in 10-12 animals. Statistically significant differences between the values obtained in BD-1063- and saline-injected mice: (A) ***p* < 0.01 (two-way ANOVA followed by Bonferroni test); (B) ***p* < 0.01 (Student *t* test).
**Figure 4****:** Effects of the s.c. administration of BD-1063 (16-64 mg/kg), Example 1 (compound 63·HCl (32-128 mg/kg), NE-100 (16-64 mg/kg), or saline (0) on the pain-related behaviors evoked by i.p. administration of cyclophosphamide (300 mg/kg) in wild-type (WT) and σ₁ receptor knockout (KO) mice. The drug or saline was injected at 120 min after the administration of cyclophosphamide. Behavioral pain responses were recorded at 30 min intervals over the 150-240 min observation period after the cyclophosphamide injection. Each bar and vertical line represents the mean ± SEM of values obtained in 10-12 animals. Statistically significant differences between the values obtained in drug- and saline-injected mice:***p* < 0.01 (one-way ANOVA followed by Bonferroni test).

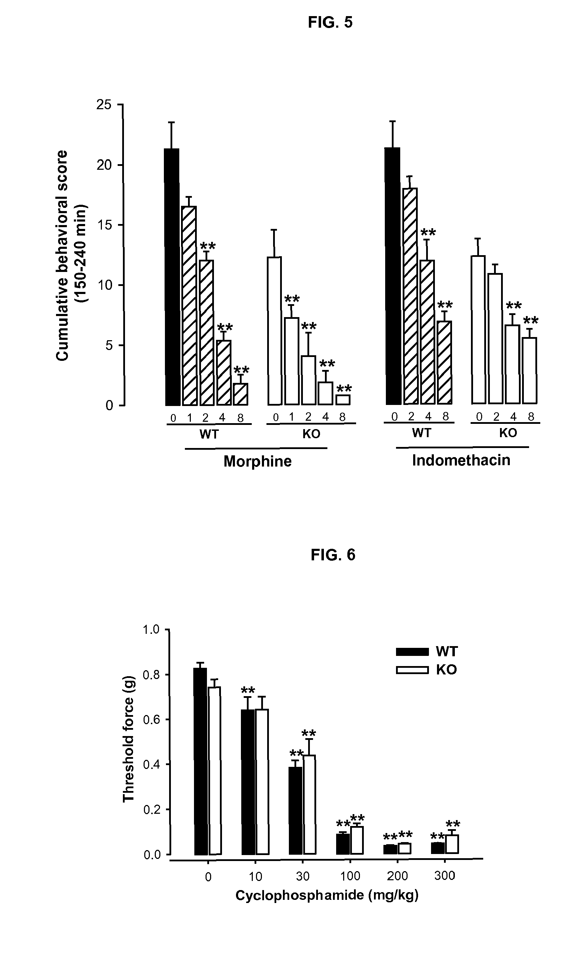

**Figure 5****:** Effects of the s.c. administration of morphine (1-8 mg/kg) or indomethacin (2-8 mg/kg) on the pain-related behaviors evoked by i.p. administration of cyclophosphamide (300 mg/kg) in wild-type (WT) and σ₁ receptor knockout (KO) mice. The drug or saline was injected at 120 min after the administration of cyclophosphamide. Behavioral pain responses were recorded at 30 min intervals over the 150-240 min observation period after the cyclophosphamide injection. Each point and vertical line represents the mean ± SEM of values obtained in 10-12 animals. Statistically significant differences between the values obtained in drug- and vehicle-injected mice: ***p* < 0.01 (one-way ANOVA followed by Bonferroni test).
**Figure 6****:** Referred mechanical hyperalgesia induced by i.p. administration of different doses of cyclophosphamide (10-300 mg/kg) or its solvent (0) in wild-type (WT) and σ₁ receptor knockout (KO) mice. The referred mechanical hyperalgesia (evaluated by stimulation of the abdomen with von Frey filaments) was measured at 240 min after the cyclophosphamide injection. Each bar and vertical line represents the mean ± SEM of values obtained in 10-12 animals. Statistically significant differences between the values obtained in cyclophosphamide- and solvent-treated animals: ***p* < 0.01 (two-way ANOVA followed by Bonferroni test).

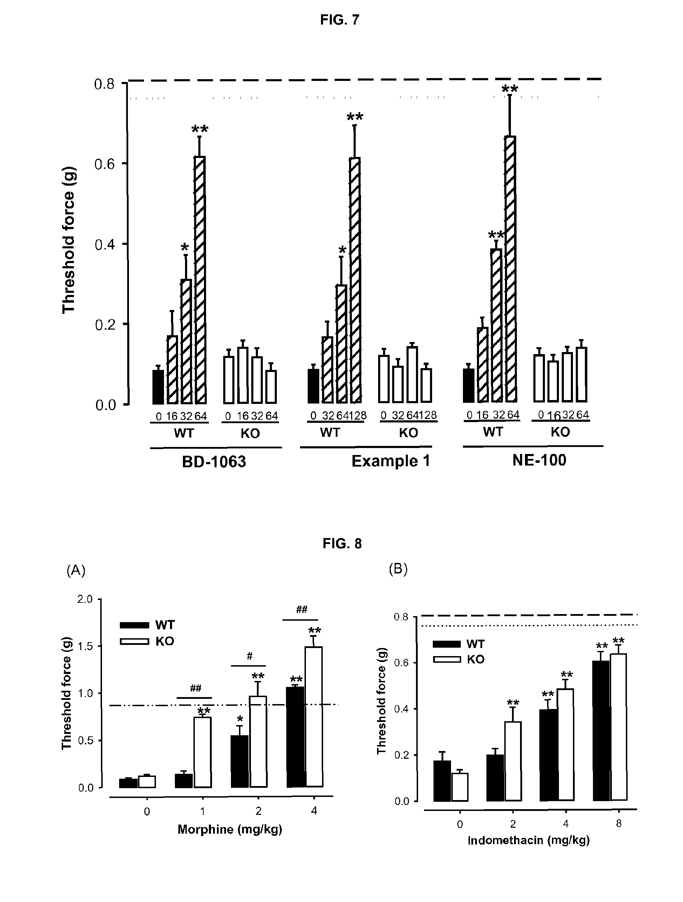

**Figure 7****:** Effects of the s.c. administration of BD-1063 (16-64 mg/kg), Example 1 (32-128 mg/kg), NE-100 (16-64 mg/kg), or saline (0) on the referred mechanical hyperalgesia induced by i.p. administration of cyclophosphamide (100 mg/kg) in wild-type (WT) and σ₁ receptor knockout (KO) mice. The drug or saline was injected at 120 min after the administration of cyclophosphamide or its solvent. The referred mechanical hyperalgesia (evaluated by stimulation of the abdomen with von Frey filaments) was measured at 240 min after the cyclophosphamide injection. Each bar and vertical line represents the mean ± SEM of values obtained in 10-12 animals. The dashed and dotted lines indicate the 50% threshold force in cyclophosphamide solvent-treated WT and KO mice, respectively. Statistically significant differences between the values obtained in drug- and saline-injected mice: **p* < 0.05; ***p* < 0.01 (one-way ANOVA followed by Bonferroni test).
**Figure 8****:** Effects of the s.c. administration of morphine (1-4 mg/kg) (A) and indomethacin (2-8 mg/kg) (B) on the referred mechanical hyperalgesia evoked by i.p. administration of cyclophosphamide (100 mg/kg) in wild-type (WT) and σ₁ receptor knockout (KO) mice. The drug or saline was injected at 120 min after the administration of cyclophosphamide or its solvent. The referred mechanical hyperalgesia (evaluated by stimulation of the abdomen with von Frey filaments) was measured at 240 min after the cyclophosphamide injection. Each bar and vertical line represents the mean ± SEM of values obtained in 10-12 animals. The dashed and dotted lines indicate the 50% threshold force in cyclophosphamide solvent-treated WT and KO mice, respectively. Note that the higher doses of morphine increase the mechanical threshold to above the control value (i.e., exert analgesic effects). Statistically significant differences between the values obtained in drug-and vehicle-injected mice: **p* < 0.05; ***p* < 0.01; and between the values obtained in wild-type and σ₁ knockout animals at the same dose of drug: ^{#}*p* < 0.05; ^{##}*p* < 0.01 (two-way ANOVA followed by Bonferroni test).

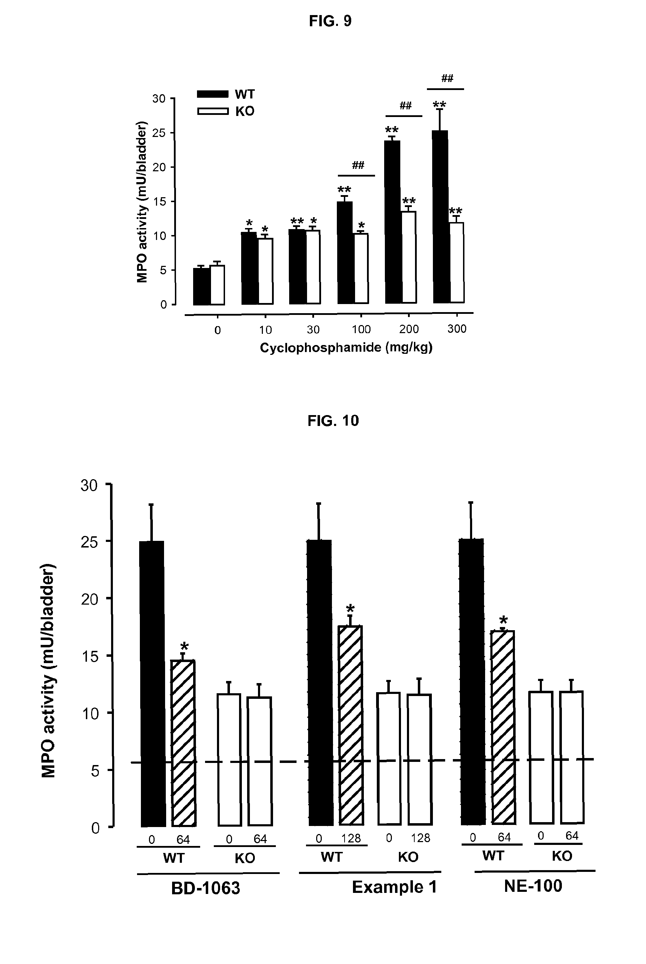

**Figure 9****:** Changes in myeloperoxidase activity (MPO) induced by i.p. administration of different doses of cyclophosphamide (10-300 mg/kg) or its solvent (0) in urinary bladder of wild-type (WT) and σ₁ receptor knockout (KO) mice. The bladder tissues were removed five hours after the injection of cyclophosphamide. Each bar and vertical line represents the mean ± SEM of values obtained in 5-7 animals. Statistically significant differences between the values obtained in cyclophosphamide- and solvent-treated animals: **p* < 0.05; ***p* < 0.01; and between the values obtained in wild-type and σ₁ knockout animals at the same dose of cyclophosphamide: ^{##}*p* < 0.01 (two-way ANOVA followed by Bonferroni test).
**Figure 10****:** Effects of the s.c. administration of BD-1063 (64 mg/kg), Example 1 (Compound 63·HCl, 128 mg/kg), NE-100 (64 mg/kg), or saline (0) on myeloperoxidase activity (MPO) induced by i.p. administration of cyclophosphamide (300 mg/kg) in wild-type (WT) and σ₁ receptor knockout (KO) mice. The drug or saline was injected at 120 min after the administration of cyclophosphamide. The bladder tissues were removed five hours after the injection of cyclophosphamide. Each bar and vertical line represents the mean ± SEM of values obtained in 5-7 animals. The dashed line indicates the MPO activity in naïve animals without any injection. Statistically significant differences between the values obtained in drug-and saline-injected mice: **p* < 0.05 (*t*-student test).

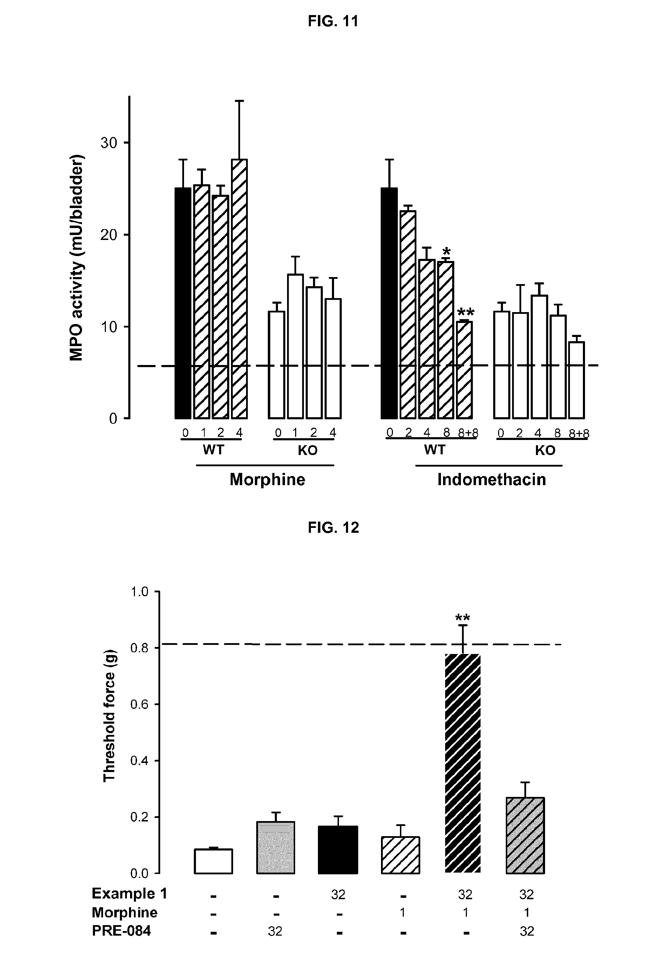

**Figure 11****:** Effects of the s.c. administration of morphine (1-4 mg/kg) and indomethacin (2-8 mg/kg) on myeloperoxidase activity (MPO) induced by i.p. administration of cyclophosphamide (300 mg/kg) in wild-type (WT) and σ₁ receptor knockout (KO) mice. The drug or saline was injected at 120 min after the administration of cyclophosphamide or its solvent. The bladder tissues were removed five hours after the injection of cyclophosphamide. Each bar and vertical line represents the mean ± SEM of values obtained in 5-7 animals. The dashed line indicates the MPO activity in naïve animals without any injection. Note that one group of mice (8+8) were treated with 8 mg/kg of indomethacin twice (30 min before and 120 min after the administration of cyclophosphamide). Statistically significant differences between the values obtained in drug- and vehicle-injected mice: **p* < 0.05; ***p* < 0.01 (one-way ANOVA followed by Bonferroni test).
**Figure 12****:** Effects of the s.c. administration of Example 1 (Compound 63·HCl. 32 mg/kg) and morphine (1 mg/kg) and its association with PRE-084 (32 mg/kg) on the referred mechanical hyperalgesia evoked by i.p. administration of cyclophosphamide (100 mg/kg) in wild-type mice. The drug or saline administered alone was injected at 120 min after the administration of cyclophosphamide or its solvent. In the association experiments, Example 1 or saline was administered 5 min before morphine or saline and 5 min after PRE-084 or saline. The referred mechanical hyperalgesia (evaluated by stimulation of the abdomen with von Frey filaments) was measured at 240 min after the cyclophosphamide injection. Each bar and vertical line represent the mean ± SEM of the values obtained in 8-10 animals. The dashed line indicates the 50% threshold force in cyclophosphamide solvent-treated mice. Statistically significant differences between the values obtained in Example 1 + morphine-injected mice and the rest of the groups: **p < 0.01 (one-way ANOVA followed by Bonferroni test).

### DETAILED DESCRIPTION OF THE INVENTION

In the context of the present invention, the following terms have the meaning detailed below.

"Alkyl" refers to a straight or branched hydrocarbon chain radical containing no unsaturation, and which is attached to the rest of the molecule by a single bond. Typical alkyl groups have from 1 to about 12, 1 to about 8, or 1 to about 6 carbon atoms, e. g., methyl, ethyl, n-propyl, i-propyl, n-butyl, t-butyl, n-pentyl, etc. If substituted by cycloalkyl, it corresponds to a "cycloalkylalkyl" radical, such as cyclopropyl methyl. If substituted by aryl, it corresponds to an "arylalkyl" radical, such as benzyl, benzhydryl or phenethyl. If substituted by heterocyclyl, it corresponds to a "heterocyclylalkyl" radical.

"Alkenyl" refers to a straight or branched hydrocarbon chain radical containing at least two carbon atoms and at least one unsaturation, and which is attached to the rest of the molecule by a single bond. Typical alkenyl radicals have from 2 to about 12, 2 to about 8 or 2 to about 6 carbon atoms. In a particular embodiment, the alkenyl group is vinyl, 1-methyl-ethenyl, 1-propenyl, 2-propenyl, or butenyl.

"Alkynyl" refers to a straight or branched hydrocarbon chain radical containing at least two carbon atoms and at least one carbon-carbon triple bond, and which is attached to the rest of the molecule by a single bond. Typical alkynyl radicals have from 2 to about 12, 2 to about 8 or 2 to about 6 carbon atoms. In a particular embodiment, the alkynyl group is ethynyl, propynyl (e.g. 1-propynyl, 2-propynyl), or butynyl (e.g. 1-butynyl, 2-butynyl, 3-butynyl).

"Cycloalkyl" refers to an alicyclic hydrocarbon. Typical cycloalkyl radicals contain from 1 to 4 separated and/or fused rings and from 3 to about 18 carbon atoms, preferably from 3 to 10 carbon atoms, such as cyclopropyl, cyclohexyl or adamantyl. In a particular embodiment, the cycloalkyl radical contains from 3 to about 6 carbon atoms.

"Aryl" refers to single and multiple ring radicals, including multiple ring radicals that contain separate and/or fused aryl groups. Typical aryl groups contain from 1 to 3 separated and/or fused rings and from 6 to about 18 carbon ring atoms, preferably from 6 to about 14 carbon ring atoms, such as phenyl, naphthyl (e.g. 2-naphthyl), biphenyl, indenyl, fenanthryl or anthracyl radical.

"Heterocyclyl" refers to a stable, typically 3- to 18-membered, ring radical which consists of carbon atoms and from one to five heteroatoms selected from the group consisting of nitrogen, oxygen, and sulfur, preferably a 4- to 8-membered ring with one or more heteroatoms, more preferably a 5- or 6-membered ring with one or more heteroatoms. It may be aromatic or not aromatic. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized ; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran, coumarine, morpholine; pyrrole, pyrazole, oxazole, isoxazole, triazole, imidazole, etc.

"Alkoxy" refers to a radical of the formula -ORₐ where Rₐ is an alkyl radical as defined above having one or more (e.g., 1, 2, 3 or 4) oxygen linkages and typically from 1 to about 12, 1 to about 8 or 1 to about 6 carbon atoms, e. g., methoxy, ethoxy, propoxy, etc.

"Aryloxy" refers to a radical of formula -O-aryl, where aryl is as previously defined. Some examples of aryloxy compounds are -O-phenyl, -O-p-tolyl, -O-m-tolyl, - O-o-tolyl or -O-naphthyl.

"Amino" refers to a radical of the formula -NH₂, -NHRₐ or -NRₐR_{b}, optionally quaternized. In an embodiment of the invention each of Rₐ and R_{b} is independently selected from hydrogen and an alkyl radical as defined above e.g., methylamino, ethylamino, dimethylamino, diethylamino, propylamino, etc..

"Halogen", "halo" or "hal" refers to bromo, chloro, iodo or fluoro.

"Fused ring system" refers to a polycyclic ring system that contains fused rings. Typically, the fused ring system contains 2 or 3 rings and/or up to 18 ring atoms. As defined above, cycloalkyl radicals, aryl radicals and heterocyclyl radicals may form fused ring systems. Thus, fused ring system may be aromatic, partially aromatic or not aromatic and may contain heteroatoms. A spiro ring system is not a fused-polycyclic by this definition, but fused polycyclic ring systems of the invention may themselves have spiro rings attached thereto via a single ring atom of the system. Examples of fused ring systems are, but are not limited to, adamantyl, naphthyl (e.g. 2-naphthyl), indenyl, fenanthryl, anthracyl, pyrenyl, benzimidazole, benzothiazole, etc.

Unless otherwise stated specifically in the specification, all the groups may be optionally substituted, if applicable. References herein to substituted groups in the compounds of the present invention refer to the specified moiety that may be substituted at one or more (e.g., 1, 2, 3 or 4) available positions by one or more suitable groups, e. g., halogen such as fluoro, chloro, bromo and iodo ; cyano; hydroxyl ; nitro ; azido ; acyl, such as alkanoyl, e.g. a C₁₋₆ alkanoyl group, and the like; carboxamido; alkyl groups including those groups having 1 to about 12 carbon atoms or from 1 to about 6 carbon atoms and more preferably 1-3 carbon atoms; alkenyl and alkynyl groups including groups having one or more (e.g., 1, 2, 3 or 4) unsaturated linkages and from 2 to about 12 carbon or from 2 to about 6 carbon atoms; alkoxy groups having one or more (e.g., 1, 2, 3 or 4) oxygen linkages and from 1 to about 12 carbon atoms or 1 to about 6 carbon atoms; aryloxy such as phenoxy; alkylthio groups including those moieties having one or more (e.g., 1, 2, 3 or 4) thioether linkages and from 1 to about 12 carbon atoms or from 1 to about 6 carbon atoms; alkylsulfinyl groups including those moieties having one or more (e.g., 1, 2, 3 or 4) sulfinyl linkages and from 1 to about 12 carbon atoms or from 1 to about 6 carbon atoms ; alkylsulfonyl groups including those moieties having one or more (e.g., 1, 2, 3 or 4) sulfonyl linkages and from 1 to about 12 carbon atoms or from 1 to about 6 carbon atoms; aminoalkyl groups such as groups having one or more (e.g., 1, 2, 3 or 4) N atoms and from 1 to about 12 carbon atoms or from 1 to about 6 carbon atoms; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

The term "salt" must be understood as any form of a compound used in accordance with this invention in which said compound is in ionic form or is charged and coupled to a counter-ion (a cation or anion) or is in solution. This definition also includes quaternary ammonium salts and complexes of the active molecule with other molecules and ions, particularly, complexes formed via ionic interactions. The definition includes in particular physiologically acceptable salts; this term must be understood as equivalent to "pharmacologically acceptable salts" or "pharmaceutically acceptable salts".

The term "pharmaceutically acceptable salts" in the context of this invention means any salt that is tolerated physiologically (normally meaning that it is not toxic, particularly, as a result of the counter-ion) when used in an appropriate manner for a treatment, applied or used, particularly, in humans and/or mammals. These physiologically acceptable salts may be formed with cations or bases and, in the context of this invention, are understood to be salts formed by at least one compound used in accordance with the invention -normally an acid (deprotonated)- such as an anion and at least one physiologically tolerated cation, preferably inorganic, particularly when used on humans and/or mammals. Salts with alkali and alkali earth metals are preferred particularly, as well as those formed with ammonium cations (NH₄⁺). Preferred salts are those formed with (mono) or (di)sodium, (mono) or (di)potassium, magnesium or calcium. These physiologically acceptable salts may also be formed with anions or acids and, in the context of this invention, are understood as being salts formed by at least one compound used in accordance with the invention - normally protonated, for example in nitrogen - such as a cation and at least one physiologically tolerated anion, particularly when used on humans and/or mammals. This definition specifically includes in the context of this invention a salt formed by a physiologically tolerated acid, i.e. salts of a specific active compound with physiologically tolerated organic or inorganic acids - particularly when used on humans and/or mammals. Examples of this type of salts are those formed with: hydrochloric acid, hydrobromic acid, sulphuric acid, methanesulfonic acid, formic acid, acetic acid, oxalic acid, succinic acid, malic acid, tartaric acid, mandelic acid, fumaric acid, lactic acid or citric acid.

The term "solvate" in accordance with this invention should be understood as meaning any form a compound in accordance with the invention in which said compound is bonded by a non-covalent bond to another molecule (normally a polar solvent), including especially hydrates and alcoholates, like for example, methanolate. A preferred solvate is the hydrate.

Any compound that is a prodrug of a Sigma receptor ligand, in particular a prodrug of a compound of formula (I), is also within the scope of the invention. The term "prodrug" is used in its broadest sense and encompasses those derivatives that are converted in vivo to the compounds of the invention. Examples of prodrugs include, but are not limited to, derivatives and metabolites of the compounds of formula (I) that include biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Preferably, prodrugs of compounds with carboxyl functional groups are the lower alkyl esters of the carboxylic acid. The carboxylate esters are conveniently formed by esterifying any of the carboxylic acid moieties present on the molecule. Prodrugs can typically be prepared using well-known methods, such as those described by Burger "Medicinal Chemistry and Drug Discovery 6th ed. (Donald J. Abraham ed., 2001, Wiley) and "Design and Applications of Prodrugs" (H. Bundgaard ed., 1985, Harwood Academic Publishers).

Any compound referred to herein is intended to represent such specific compound as well as certain variations or forms. In particular, compounds referred to herein may have asymmetric centres and therefore exist in different enantiomeric or diastereomeric forms. Thus, any given compound referred to herein is intended to represent any one of a racemate, one or more enantiomeric forms, one or more diastereomeric forms, and mixtures thereof. Likewise, stereoisomerism or geometric isomerism about the double bond is also possible, therefore in some cases the molecule could exist as (E)-isomer or (Z)-isomer (trans and cis isomers). If the molecule contains several double bonds, each double bond will have its own stereoisomerism, that could be the same as, or different to, the stereoisomerism of the other double bonds of the molecule. Furthermore, compounds referred to herein may exist as atropisomers. All the stereoisomers including enantiomers, diastereoisomers, geometric isomers and atropisomers of the compounds referred to herein, and mixtures thereof, are considered within the scope of the present invention.

Furthermore, any compound referred to herein may exist as tautomers. Specifically, the term tautomer refers to one of two or more structural isomers of a compound that exist in equilibrium and are readily converted from one isomeric form to another. Common tautomeric pairs are amine-imine, amide-imidic acid, keto-enol, lactam-lactim, etc.

Unless otherwise stated, the compounds used in the invention are also meant to include isotopically-labelled forms i.e. compounds which differ only in the presence of one or more isotopically-enriched atoms. For example, compounds having the present structures except for the replacement of at least one hydrogen atom by a deuterium or tritium, or the replacement of at least one carbon by ¹³C- or ¹⁴C-enriched carbon, or the replacement of at least one nitrogen by ¹⁵N-enriched nitrogen are within the scope of this invention.

The compounds used in the invention or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts, solvates or prodrugs.

As used herein, the terms "treat", "treating" and "treatment" include the eradication, removal, reversion, alleviation, modification, or control of pain associated to IC/BPS, after the pain onset.

As used herein, the terms "prevention", "preventing", "preventive" "prevent" and "prophylaxis" refer to the capacity of a therapeutic to avoid, minimize or difficult the onset or development of pain associated to IC/BPS before its onset.

Therefore, by "treating" or "treatment" and/or "preventing" or "prevention", as a whole, is meant at least a suppression or an amelioration of the symptoms associated with the condition afflicting the subject, where suppression and amelioration are used in a broad sense to refer to at least a reduction in the magnitude of a parameter, e.g., symptom associated with the condition being treated, i.e. pain associated to IC/BPS. As such, the method of the present invention also includes situations where the condition is completely inhibited, e.g., prevented from happening, or stopped, e.g., terminated, such that the subject no longer experiences the condition.

"The sigma receptor/s" as used in this application is/are well known and defined using the following citation: "this binding site represents a typical protein different from opioid, NMDA, dopaminergic, and other known neurotransmitter or hormone receptor families" (G. Ronsisvalle et al. Pure Appl. Chem. 73, 1499-1509 (2001)). Pharmacological data based on ligand binding studies, anatomical distribution and biochemical features distinguish at least two subtypes of sigma (σ) receptors (R. Quiron et al., Trends Pharmacol. Sci. 13, 85-86 (1992); M.L.Leitner, Eur. J. Pharmacol. 259, 65-69 (1994); S.B. Hellewell and W.D. Bowen, Brain Res. 527, 244-253 (1990); G. Ronsisvalle et al. Pure Appl. Chem. 73, 1499-1509 (2001)). The protein sequence of the sigma-1 receptor (σ1) is known in the art (e.g. Prasad, P.D. et al., J. Neurochem. 70 (2), 443-451 (1998)). They show a very high affinity to various analgesics (e.g. pentazocine).

As used herein, the terms "Sigma ligand" or "Sigma receptor ligand" refer to any "compound binding to the Sigma receptor". Compounds binding to the sigma receptor are well known in the art. "Compound/s binding to the Sigma receptor" or "sigma ligand" as used in this application is/are preferably defined as a compound having an IC₅₀ value of ≤ 5000 nM, more preferably ≤ 1000 nM, more preferably ≤ 500 nM on the sigma receptor. More preferably, the IC₅₀ value is ≤ 250 nM. More preferably, the IC₅₀ value is ≤ 100 nM. Most preferably, the IC₅₀ value is ≤ 50 nM. The half maximal inhibitory concentration (IC₅₀) is a measure of the effectiveness of a compound in inhibiting biological or biochemical function. The IC₅₀ is the concentration of competing ligand which displaces 50% of the specific binding of the radioligand. Additionally, the wording "Compound/s binding to the sigma receptor", as used in the present application is preferably defined as having at least ≥50% displacement using 10 nM radioligand specific for the sigma receptor (e.g. preferably [³H]-(+) pentazocine) whereby the sigma receptor may be any sigma receptor subtype. Preferably, said compounds bind to the sigma-1 receptor subtype.

Further, said compounds binding to the sigma receptor as defined herein, may be antagonists, inverse agonists, agonists, partial antagonists and/or partial agonists. The sigma ligand according to the present invention is preferably a sigma receptor antagonist in the form of a (neutral) antagonist, an inverse agonist or a partial antagonist.

In a preferred embodiment of the invention the Sigma receptor ligand is a selective Sigma-1 antagonist, preferably in the form of a (neutral) antagonist, an inverse agonist or a partial antagonist, more preferably a selective Sigma-1 (neutral) antagonist.

An "agonist" is defined as a compound that binds to a receptor and has an intrinsic effect, and thus, increases the basal activity of a receptor when it contacts the receptor.

An "antagonist" is defined as a compound that competes with an agonist or inverse agonist for binding to a receptor, thereby blocking the action of an agonist or inverse agonist on the receptor. However, an antagonist (also known as a "neutral" antagonist) has no effect on constitutive receptor activity. Antagonists mediate their effects by binding to the active site or to allosteric sites on receptors, or they may interact at unique binding sites not normally involved in the biological regulation of the receptor's activity. Antagonist activity may be reversible or irreversible depending on the longevity of the antagonist-receptor complex, which, in turn, depends on the nature of antagonist receptor binding.

A "partial antagonist" is defined as a compound that binds to the receptor and generates an antagonist response; however, a partial antagonist does not generate the full antagonist response. Partial antagonists are weak antagonists, thereby blocking partially the action of an agonist or inverse agonist on the receptor.

An "inverse agonist" is defined as a compound that produces an effect opposite to that of the agonist by occupying the same receptor and, thus, decreases the basal activity of a receptor (i.e., signalling mediated by the receptor). Such compounds are also known as negative antagonists. An inverse agonist is a ligand for a receptor that causes the receptor to adopt an inactive state relative to a basal state occurring in the absence of any ligand. Thus, while an antagonist can inhibit the activity of an agonist, an inverse agonist is a ligand that can alter the conformation of the receptor in the absence of an agonist.

Although there are many known uses for sigma ligands, such as antipsychotic drugs, anxiolytics, antidepressants, stroke treatment, antiepileptic drugs and many other indications, including anti-migraine and general pain, there is no mention in the art of these compounds as useful for the treatment of of pain associated to IC/BPS.
Table 1 lists some sigma ligands known in the art (i.e. having an IC₅₀ ≤ 5000 nM). Some of these compounds may bind to the sigma-1 and/or to the sigma-2 receptor. These sigma ligands also include their respective salts, bases, and acids.

**Table 1**

| | |
|---|---|
| Acetophenazine Maleate | Fluphenazine Decanoate DiHCl |
| Alverine | Fluphenazine Enanthate DiHCl |
| Aminobenztropine | Fluphenazine HCl |
| Amorolfine HCl | Fluphenazine N-Mustard DiHCl |
| AN2/AVex-73; AE-37; ANAVEX 2-73; N-(2,2-Diphenyltetrahydrofuran-3-ylmethyl)-N,N-dimethylamine | Fluspidine |
| Anileridine | Fentanyl |
| BD-1063 | GBR-12935 DiHCl |
| BD-1008 | HEAT HCl |
| BD-1047 | I-693,403 |
| Benproperine Phosphate | Ifenprodil Tartrate |
| Benztropine Mesylate | Igmesine |
| Bromhexine HCl | LR132 |
| Bromperidol | Lobeline HCl |
| Carbetapentane Citrate | Lomerizine diHCl |
| Cinnarizine | Loperamide |
| Cis(Z)-Flupentixol DiHCl | Mebeverine |
| Clobenztropine | Naftifine |
| Clorgyline HCl | NE-100 |
| Cutamesine hydrochloride | Opipramol |
| Cyclobenzaprine HCl | Oxybutynin |
| Dicyclomine HCl | Pirlindole |
| Dimemorphan | Perphenazine |
| Dextromethorphan | Sertraline |
| Ditolylguanidine | Sufentanyl |
| Duloxetine | Terbinafine HCl |
| Dibenzheptoprine | Trifluoperazine HCl |
| Donepezil | Trifluperidol HCl |
| Eliprodil | Trimeprazine Hemi-L-Tartrate |
| Fluvoxamine | Vanoxerine |
| Flunarizine diHCl | Xylazine |

Preferably, the table above includes also haloperidol, haloperidol metabolite I (4-(4-chlorophenyl)-4-hydroxypiperidine) and haloperidol metabolite II (4-(4-chlorophenyl)-α-(4-fluorophenyl)-4-hydroxy-1-piperidinebutanol) also called reduced haloperidol Studies performed in rodent brain membranes and human neuroblastoma cells showed that metabolites I and II of haloperidol bind to σ1 receptors with less affinity than haloperidol, but show much lower (metabolite II) or no affinity (metabolite I) for D2 receptors. Reduced haloperidol or metabolite II, an active metabolite of haloperidol that is produced in humans, shows a high affinity (in the low nanomolar range) for sigma-1 receptors, and produces an irreversible blockade of sigma-1 receptors both in experimental animals and human cells.

In a preferred embodiment, the Sigma receptor ligand in the context of the present invention has the general formula (I) as depicted above.

In a preferred embodiment, R₁ in the compounds of formula (I) is selected from H, -COR₈, and substituted or unsubstituted alkyl. More preferably, R₁ is selected from H, methyl and acetyl. A more preferred embodiment is when R₁ is H.

In another preferred embodiment, R₂ in the compounds of formula (I) represents H or substituted or unsubstituted alkyl, more preferably methyl.

In yet another preferred embodiment of the invention, R₃ and R₄ in the compounds of formula (I) are situated in the meta and para positions of the phenyl group, and preferably, they are selected independently from halogen and substituted or unsubstituted alkyl.

In an especially preferred embodiment of the invention, in the compounds of formula (I) both R₃ and R₄ together with the phenyl group form an optionally substituted fused ring system. More preferably, said fused ring system is selected from a substituted or unsubstituted fused aryl group and a substituted or unsubstituted aromatic or partially aromatic fused heterocyclyl group. Said fused ring system preferably contains two rings and/or from 9 to about 18 ring atoms, more preferably 9 or 10 ring atoms. Even more preferably, the fused ring system is naphthyl, especially a 2-naphthyl ring system.

Also in the compounds of formula (I), embodiments where n is selected from 2, 3, 4 are preferred in the context of the present invention, more preferably n is 2.

Finally, in another embodiment it is preferred in the compounds of formula (I) that R₅ and R₆ are, each independently, C₁₋₆ alkyl, or together with the nitrogen atom to which they are attached form a substituted or unsubstituted heterocyclyl group, in particular a group chosen among morpholinyl, piperidinyl, and pyrrolidinyl group. More preferably, R₅ and R₆ together form a morpholine-4-yl group.

In additional preferred embodiments, the preferences described above for the different substituents are combined. The present invention is also directed to such combinations of preferred substitutions in the formula (I) above.

In preferred variants of the invention, the Sigma ligand of general formula (I) is selected from:
[1] 4-{2-(1-(3,4-dichlorophenyl)-5-methyl-1H pyrazol-3-yloxy)ethyl} morpholine,
[2] 2-[1-(3,4-Dichlorophenyl)-5-methyl-1H-pyrazol-3-yloxy]-N,N-diethylethanamine,
[3] 1-(3,4-Dichlorophenyl)-5-methyl-3-[2-(pyrrolidin-1-yl)ethoxy]-1H-pyrazole,
[4] 1-(3,4-Dichlorophenyl)-5-methyl-3-[3-(pyrrolidin-1-yl)propoxy]-1H-pyrazole,
[5] 1-{2-[1-(3,4-Dichiorophenyl)-5-methyl-1H-pyrazol-3-yloxy]ethyl}piperidine,
[6] 1-{2-[1-(3,4-dichlorophenyl)-5-methyl-1H-pyrazol-3-yloxy]ethyl}-1H-imidazole,
[7] 3-{1-[2-(1-(3,4-Dichlorophenyl)-5-methyl-1H-pyrazol-3-yloxy)ethyl]piperidin-4-yl}-3H-imidazo[4,5-b]pyridine,
[8]1-{2-[1-(3,4-Dichlorophenyl)-5-methyl-1H-pyrazol-3-yloxy]ethyl}-4-methylpiperazine,
[9] Ethyl 4-{2-[1-(3,4-dichiorophenyl)-5-methyl-1H-pyrazol-3-yioxy]ethyl} piperazine carboxylate,
[10] 1-(4-(2-(1-(3,4-dichlorophenyl)-5-methyl-1H-pyrazol-3-yloxy)ethyl)piperazin-1-yl)ethanone,
[11] 4-{2-[1-(4-Methoxyphenyl)-5-methyl-1H-pyrazol-3-yloxy]ethyl}morpholine,
[12] 1-(4-Methoxyphenyl)-5-methyl-3-[2-(pyrrolidin-1-yl)ethoxy]-1H-pyrazole,
[13] 1-(4-Methoxyphenyl)-5-methyl-3-[3-(pyrrolidin-1-yl)propoxy]-1H-pyrazole,
[14] 1-[2-(1-(4-Methoxyphenyl)-5-methyl-1H-pyrazol-3-yloxy)ethyl]piperidine,
[15]1-{2-[1-(4-Methoxyphenyl)-5-methyl-1H-pyrazol-3-yloxy]ethyl}-1H-imidazole,
[16] 4-{2-[1-(3,4-Dichlorophenyl)-5-phenyl-1H-pyrazol-3-yloxy]ethyl} morpholine,
[17] 1-(3,4-Dichlorophenyl)-5-phenyl-3-[2-(pyrrolidin-1-yl)ethoxy]-1H-pyrazole,
[18] 1-(3,4-Dichlorophenyl)-5-phenyl-3-[3-(pyrrolidin-1-yl)propoxy]-1H-pyrazole,
[19] 1-{2-[1-(3,4-Dichlorophenyl)-5-phenyl-1H-pyrazol-3-yloxy]ethyl}piperidine,
[20] 1-{2-[1-(3,4-Dichiorophenyl)-5-phenyl-1H-pyrazol-3-yloxy]ethyl}-1H-imidazole,
[21]2-{2-[1-(3,4-dichlorophenyl)-5-phenyl-1H-pyrazol-3-yloxy]ethyl}-1,2,3,4-tetrahydroisoquinoline,
[22] 4-{4-[1-(3,4-Dichlorophenyl)-5-methyl-1H-pyrazol-3-yloxy]butyl} morpholine,
[23] 1-(3,4-Dichlorophenyl)-5-methyl-3-[4-(pyrrolidin-1-yl)butoxy]-1H-pyrazole,
[24] 1-{4-[1-(3,4-Dichlorophenyl)-5-methyl-1H-pyrazol-3-yloxy]butyl}piperidine,
[25]1-{4-[1-(3,4-Dichlorophenyl)-5-methyl-1H-pyrazol-3-yloxy]butyl}-4-methylpiperazine,
[26] 1-{4-[1-(3,4-Dichlorophenyl)-5-methyl-1H-pyrazol-3-yioxy]butyl}-1H-imidazole,
[27] 4-[1-(3,4-Dichlorophenyl)-5-methyl-1H-pyrazol-3-yloxy]-N,N-diethylbutan-1-amine,
[28]1-{4-[1-(3,4-dichlorophenyl)-5-methyl-1H-pyrazol-3-yloxy]butyl}-4-phenylpiperidine,
[29] 1-{4-[1-(3,4-dichlorophenyl)-5-methyl-1H-pyrazol-3-yloxy]butyl}-6,7-dihydro-1H-indol-4(5H)-one,
[30] 2-{4-[1-(3,4-dichlorophenyl)-5-methyl-1H-pyrazol-3-yloxy]butyl}-1,2,3,4-tetrahydroisoquinoline,
[31] 4-{2-[1-(3,4-dichlorophenyl)-5-isopropyl-1H-pyrazol-3-yloxy]ethyl} morpholine,
[32]2-[1-(3,4-Dichlorophenyl)-5-isopropyl-1 H-pyrazol-3-yloxy]-N,N-diethylethanamine,
[33] 1-(3,4-Dichlorophenyl)-5-isopropyl-3-[2-(pyrrolidin-1-yl)ethoxy]-1H-pyrazole,
[34] 1-(3,4-Dichlorophenyl)-5-isopropyl-3-[3-(pyrrolidin-1-yl)propoxy]-1H-pyrazole,
[35] 1-{2-[1-(3,4-Dichlorophenyl)-5-isopropyl-1H-pyrazol-3-yloxy]ethyl} piperidine,
[36] 2-{2-[1-(3,4-dichlorophenyl)-5-isopropyl-1H-pyrazol-3-yloxy]ethyl}-1,2,3,4-tetrahydroiso quinoline,
[37] 4-{2-[1-(3,4-dichlorophenyl)-1H-pyrazol-3-yloxy]ethyl}morpholine,
[38] 2-[1-(3,4-dichlorophenyl)-1H-pyrazol-3-yloxy] N,N-diethylethanamine,
[39] 1-(3,4-dichlorophenyl)-3-[2-(pyrrolidin-1-yl)ethoxy]-1H-pyrazole,
[40] 1-{2-[1-(3,4-dichlorophenyl)-1H-pyrazol-3-yloxy]ethyl}piperidine,
[41] 1-(3,4-dichlorophenyl)-3-[3-(pyrrolidin-1-yl)propoxy]-1H-pyrazole,
[42]1-{2-[1-(3,4-Dichlorophenyl)-5-methyl-1H-pyrazol-3-yloxy]ethyl}piperazine,
[43] 1-{2-[1-(3,4-Dichlorophenyl)-5-methyl-1H-pyrazol-3-yloxy]ethyl}pyrrolidin-3-amine,
[44]4-{2-[1-(3,4-Dichlorophenyl)-4,5-dimethyl-1H-pyrazol-3-yloxy]ethyl}morpholine,
[46]2-[1-(3,4-Dichlorophenyl)-4,5-dimethyl-1H-pyrazol-3-yloxy]-N,N-diethylethanamine,
[47] 1-(3,4-Dichlorophenyl)-4,5-dimethyl-3-[2-(pyrrolidin-1-yl)ethoxy]-1H-pyrazole,
[48] 1-(3,4-Dichlorophenyl)-4,5-dimethyl-3-[3-(pyrrolidin-1-yl)propoxy]-1H-pyrazole,
[49] 1-{2-[1-(3,4-Dichlorophenyl)-4,5-dimethyl-1H-pyrazol-3-yloxy]ethyl} piperidine,
[50] 4-{4-[1-(3,4-dichlorophenyl)-1H-pyrazol-3-yloxy]butyl}morpholine,
[51]((25,6R)-4-{4-[1-(3,4-dichlorophenyl)-1H-pyrazol-3-yloxy]butyl}-2,6-dimethylmorpholine,
[52] 1-{4-[1-(3,4-Dichlorophenyl)-1H-pyrazol-3-yloxy]butyl}piperidine,
[53] 1-(3,4-Dichlorophenyl)-3-[4-(pyrrolidin-1-yl)butoxy]-1H-pyrazole,
[55] 4-[1-(3,4-dichlorophenyl)-1H-pyrazol-3-yloxy]-N,N-diethylbutan-1-amine,
[56] N-benzyl-4-[1-(3,4-dichlorophenyl)-1H-pyrazol-3-yloxy]-N-methylbutan-1-amine,
[57]4-[1-(3,4-dichlorophenyl)-1H-pyrazol-3-yloxy]-N-(2-methoxyethyl)-N-methylbutan-1-amine,
[58] 4-{4-[1-(3,4-dichlorophenyl)-1H-pyrazol-3-yloxy]butyl}thiomorpholine,
[59]1-[1-(3,4-Dichlorophenyl)-5-methyl-3-(2-morpholinoethoxy)-1H-pyrazol-4-yl]ethanone,
[60] 1-{1-(3,4-dichlorophenyl)-5-methyl-3-[2-(pyrrolidin-1-yl)ethoxy]-1H-pyrazol-4-yl}ethanone,
[61] 1-{1-(3,4-dichlorophenyl)-5-methyl-3-[2-(piperidin-1-yl)ethoxy]-1H-pyrazol-4-yl}ethanone,
[62] 1-{1-(3,4-dichlorophenyl)-3-[2-(diethylamino)ethoxy]-5-methyl-1H-pyrazol-4-yl}ethanone,
[63] 4-{2-[5-Methyl-1-(naphthalen-2-yl)-1H-pyrazol-3-yloxy]ethyl}morpholine,
[64] N,N-Diethyl-2-[5-methyl-1-(naphthalen-2-yl)-1H-pyrazol-3-yloxy] ethanamine,
[65] 1-{2-[5-Methyl-1-(naphthalen-2-yl)-1H-pyrazol-3-yloxy]ethyl}piperidine,
[66] 5-Methyl-1-(naphthalen-2-yl)-3-[2-(pyrrolidin-1-yl)ethoxy]-1H-pyrazole,
and their pharmaceutically acceptable salts, solvates or prodrugs

In a preferred variant of the invention, the Sigma ligand of general formula (I) is 4-{2-[5-methyl-1-(naphthalen-2-yl)-1H-pyrazol-3-yloxy]ethyl} morpholine or a salt thereof.

Preferably, the compound of general formula (I) used is 4-{2-[5-methyl-1-(naphthalen-2-yl)-1H-pyrazol-3-yloxy]ethyl]morpholine hydrochloride.

These particular compounds are designated in the examples of the present invention as compound 63 and compound 63·HCl.

The compounds of general formula (I) and their salts or solvates can be prepared as disclosed in the previous application WO2006/021462.

A further aspect of the present invention relates to a medicament or composition in different pharmaceutical forms comprising at least one Sigma receptor ligand (preferably a compound of formula (I)) and at least one pharmaceutically acceptable excipient for use in the treatment and/or prevention of pain associated to IC/BPS.

The composition can be used with at least another drug to provide a combination therapy. This other drug or drugs may be part of the same composition, or may be provided as a separate composition and can be administered at the same time or at different times.

According to a particular embodiment, the pharmaceutical composition of the invention refers to a combination of at least one Sigma receptor ligand (preferably a compound of formula (I)) and at least one drug currently used for the pain associated to IC/BPS therapy. Drugs currently used for IC/BPS associated pain include:
- oral drugs such as bladder mucosal protectors (pentosan polysulphate (Elmiron)); anti-allergics as antihistaminics (H1 blockers (hydroxyzine hydrochloride) as well as H2 blockers (Cimetidine)); leukotriene-D4 receptor antagonist montelukast; pain modulators as trycyclic antidepressants (Amitriptyline) or anticonvulsants (gabapentin); hormone modulators (Leuprolide acetate); anti-inflammatory agents as anti-TNF; narcotics; pain relief agents (opioids, Morphine, Tramadol); immunosuppressive agents (Prednisone, Triamcinolone); L-arginine; Oxybutynin; or Tolterodine;
- intravesical drugs such as pain modulators (Dimethylsulfoxide or Bacillus Calmette-Guerin (BCG)), bladder mucosal protectors (Hyaluronic acid), or Chondroitin sulphate; and
- complementary drugs that utilize anti-inflammatory, neural, anesthesic and behavioural agents.

Further, the Sigma receptor ligand may be administered in conjunction with a surgical treatment.

The combination may be formulated for its simultaneous, separate or sequential administration, with at least a pharmaceutically acceptable excipient. This has the implication that the combination of the Sigma receptor ligand and the other drug (such as one drug currently used for IC/BPS) may be administered:
a) As a combination that is being part of the same medicament composition, both being then administered always simultaneously.
b) As a combination of two units, each with one of them giving rise to the possibility of simultaneous, sequential or separate administration. In a particular embodiment, the Sigma receptor ligand is independently administered from the other drug (i.e in two units) but at the same time. In another particular embodiment, the Sigma receptor ligand is administered first, and then the other drug is separately or sequentially administered. In yet another particular embodiment, the other drug is administered first, and then the Sigma receptor ligand is administered, separately or sequentially, as defined.

In a preferred embodiment of the present invention the combination comprises at least one Sigma receptor ligand and at least one opioid. In a more preferably embodiment the combination comprises 4-{2-[5-Methyl-1-(naphthalen-2-yl)-1H-pyrazol-3-yloxy]ethyl}morpholine hydrochloride and morphine.

The term "excipient" refers to components of a drug compound other than the active ingredient (definition obtained from the European Medicines Agency- EMA). They preferably include a "carrier, adjuvant and/or vehicle". Carriers are forms to which substances are incorporated to improve the delivery and the effectiveness of drugs. Drug carriers are used in drug-delivery systems such as the controlled-release technology to prolong in vivo drug actions, decrease drug metabolism, and reduce drug toxicity. Carriers are also used in designs to increase the effectiveness of drug delivery to the target sites of pharmacological actions (U.S. National Library of Medicine. National Institutes of Health). Adjuvant is a substance added to a drug product formulation that affects the action of the active ingredient in a predictable way. Vehicle is an excipient or a substance, preferably without therapeutic action, used as a medium to give bulk for the administration of medicines (Stedman's Medical Spellchecker, © 2006 Lippincott Williams & Wilkins). Such pharmaceutical carriers, adjuvants or vehicles can be sterile liquids, such as water and oils, including those of petroleum, animal, vegetable or synthetic origin, such as peanut oil, soybean oil, mineral oil, sesame oil and the like, excipients, disgregants, wetting agents or diluents. Suitable pharmaceutical carriers are described in "Remington's Pharmaceutical Sciences" by E.W. Martin. The selection of these excipients and the amounts to be used will depend on the form of application of the pharmaceutical composition.

The pharmaceutical compositions in accordance with the invention can be adapted in order to be administered by any route of administration, be it orally or parenterally, such as pulmonarily, nasally, rectally and/or intravenously. Therefore, the formulation in accordance with the invention may be adapted for topical or systemic application, particularly for dermal, subcutaneous, intramuscular, intra-articular, intraperitoneal, pulmonary, buccal, sublingual, nasal, percutaneous, vaginal, oral or parenteral application. The preferred form of rectal application is by means of suppositories.

Suitable preparations for oral applications are tablets, pills, chewing gums, capsules, granules, drops or syrups. Suitable preparations for parenteral applications are solutions, suspensions, reconstitutable dry preparations or sprays.

The pharmaceutical composition of the invention may be formulated as deposits in dissolved form or in patches, for percutaneous application. Skin applications include ointments, gels, creams, lotions, suspensions or emulsions.

In a particular embodiment of the present invention, the pain is selected from peripheral neuropathic pain, allodynia, causalgia, hyperalgesia, hyperesthesia, hyperpathia, neuralgia, neuritis or neuropathy.

"Neuropathic pain" is defined by the IASP as "pain initiated or caused by a primary lesion or dysfunction in the nervous system" (IASP, Classification of chronic pain, 2nd Edition, IASP Press (2002), 210). For the purpose of this invention this term is to be treated as synonymous to "Neurogenic Pain" which is defined by the IASP as "pain initiated or caused by a primary lesion, dysfunction or transitory perturbation in the peripheral or central nervous system". Neuropathic pain according to this invention is restricted to the neuropathic pain resulting from a surgery.

According to the IASP "peripheral neuropathic pain" is defined as "a pain initiated or caused by a primary lesion or dysfunction in the peripheral nervous system" and "peripheral neurogenic pain" is defined as "a pain initiated or caused by a primary lesion, dysfunction or transitory perturbation in the peripheral nervous system" (IASP, Classification of chronic pain, 2nd Edition, IASP Press (2002), 213).

According to the IASP "allodynia" is defined as "a pain due to a stimulus which does not normally provoke pain" (IASP, Classification of chronic pain, 2nd Edition, IASP Press (2002), 210).

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

According to the IASP "hyperalgesia" is defined as "an increased response to a stimulus which is normally painful" (IASP, Classification of chronic pain, 2nd Edition, IASP Press (2002), 211).

According to the IASP "hyperesthesia" is defined as "increased sensitivity to stimulation, excluding the senses" (IASP, Classification of chronic pain, 2nd Edition, IASP Press (2002), 211).

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition, IASP Press (2002), 212).

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold Increased response | Stimulus and response rate may be the same or different |

According to the IASP "neuralgia" is defined as "pain in the distribution of a nerve or nerves" (IASP, Classification of chronic pain, 2nd Edition, IASP Press (2002), 212).

According to the IASP "neuritis" is defined as "inflammation of a nerve or nerves" (IASP, Classification of chronic pain, 2nd Edition, IASP Press (2002), 212).

According to the IASP "neuropathy/neuritis" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

Another aspect of the invention is a method of treatment of a patient suffering, or likely to suffer, pain associated to IC/BPS, which comprises administering to the patient in need of such a treatment or prophylaxis a therapeutically effective amount of a sigma ligand, preferably a sigma ligand of formula (I), or a pharmaceutically acceptable salt, isomer, prodrug or solvate thereof.

Generally an effective administered amount of a compound used in the invention will depend on the relative efficacy of the compound chosen, the severity of the disorder being treated, or the age, weight or mode of administration. However, active compounds will typically be administered once or more times a day, for example 1, 2, 3 or 4 times daily, with typical total daily doses in the range of from 0.1 to 500 mg/kg/day.

Having described the present invention in general terms, it will be more easily understood by reference to the following examples which are presented as an illustration and are not intended to limit the present invention.

### Examples

### Example 1: Synthesis of 4-{2-[5-Methyl-1-(naphthalen-2-yl)-1H-pyrazol-3-yloxy]ethyl} morpholine hydrochloride (compound 63·HCl)

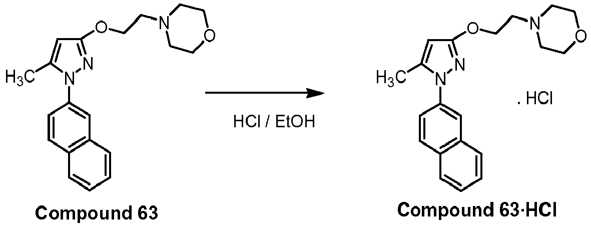

Compound 63 can be prepared as disclosed in the previous application WO2006/021462. Its hydrochloride can be obtained according the following procedure:

Compound 63 (6.39 g) was dissolved in ethanol saturated with HCl. The mixture was stirred then for some minutes and evaporated to dryness. The residue was crystallized from isopropanol. The mother liquors from the first crystallization afforded a second crystallization by concentrating. Both crystallizations taken together yielded 5.24 g (63 %) of the corresponding hydrochloride salt (m.p. = 197-199°C.)

¹H-NMR (DMSO-d₆) δ ppm: 10,85 (bs, 1H), 7,95 (m, 4H), 7,7 (dd, J=2,2, 8,8 Hz, 1 H), 7,55 (m, 2H), 5,9 (s, 1 H), 4,55 (m, 2H), 3,95 (m, 2H), 3,75 (m, 2H), 3,55-3,4 (m, 4H), 3,2 (m, 2H), 2,35 (s, 3H). HPLC purity: 99.8%

### Example 2: Role of Sigma-1 receptors in cyclophosphamide induced cystitis

Existing animal models of visceral pain in the mouse are of limited practical usefulness since they are not visceral specific. The animal model of cyclophosphamide-induced cystitis keeps inflammation features confined to the bladder and results in an attractive model to investigate the genetic and physiological bases of visceral pain as interstitial cystitis/painful bladder syndrome (IC/PBS). (Elsa Anton, 2002; Bon et al., 2003; J Urol. Sep;170(3):1008-12).

### 2.1 Materials and Methods

### 2.1.1 Animals

Experiments were performed in female wild-type (WT, Charles River, Barcelona, Spain) and σ₁ receptor knockout (σ₁-KO, Laboratorios Esteve, Barcelona, Spain) CD-1 mice weighing 25-30 g. The σ₁-KO mice were generated on a CD-1 background as previously described (Entrena et al., 2009). Animals were acclimated in our animal facilities for at least 1 week before testing, housed in colony cages in temperature and light-controlled rooms (22 ± 1 °C, lights on at 08.00 h and off at 20.00 h, air replacement every 20 min). A standard laboratory diet (Harlan Teklad Research diet, Madison, USA) and tap water were available *ad libitum* until the beginning of the experiments. Testing took place during the light phase (from 9.00 h to 15.00 h). Mice were handled in accordance with the European Communities Council Directive of 24 November 1986 (86/609/ECC), and the experimental protocol was approved by the University of Granada Research Ethics Committee.

### 2.1.2 Drugs and drug administration

The selective Sigma-1 receptor antagonists BD-1063 (1-[2-(3,4-dichlorophenyl)ethyl]-4-methylpiperazine) supplied by Tocris Cookson Ltd. (Bristol, UK), NE-100 (N, N-dipropyl-2-[4-methoxy-3-(2-phenylethoxy)phenyl]ethylamine hydrochloride), synthesized as previously reported (Nakazato et al., 1999), Example 1 (compound 63·HCl), and the selective σ₁ receptor agonist PRE-084 [2-(4-morpholinethyl)1-phenylcyclohexanecarboxylate) hydrochloride] supplied by Tocris Cookson Ltd. (Bristol, UK) were used as test compounds. Morphine hydrochloride (General Directorate of Pharmacy and Drugs, Spanish Ministry of Health) and indomethacin (Sigma-Aldrich Química S.A., Madrid, Spain) were used as opioid and non-steroidal anti-inflammatory control drugs, respectively. All drugs were dissolved in sterile physiological saline with the exception of indomethacin, which was dissolved in 5% sodium bicarbonate (Panreac Química S.L.U., Barcelona, Spain). Drug solutions were prepared immediately before the start of the experiments, and 5 ml/kg of the drug solution or its solvent was injected subcutaneously (s.c.) into the interscapular area. Cyclophosphamide (Sigma-Aldrich), which was used to induce cystitis, was dissolved in saline and injected intraperitoneally (i.p.) at the volume of 10 ml/kg. Control animals were injected with the same volume of solvents.

### 2.2 General procedures for evaluating cyclophosphamide-evoked visceral pain and referred hyperalgesia

Spontaneous pain-related behaviors and referred mechanical hyperalgesia induced by cyclophosphamide were tested following a previously described protocol, (Olivar and Laird, 1999; Laird et al., 2002; Wantuch et al., 2007) with small modifications. Mice were housed in individual transparent plastic boxes (7 x 7 x 13 cm) on an elevated platform with a wire mesh floor (small mirrors behind and below the chambers enhanced observation of the animals). After a 40-min habituation period, animals were removed from the compartments, and injected with the cyclophosphamide solution (or its solvent). The animals were immediately returned to the compartment, where they were observed for 2 min every half-hour over a 4-h observation period after the cyclophosphamide injection. The recorded pain-related behaviors were coded according to the following scale: 0 = normal, 1 = piloerection, 2 = strong piloerection, 3 = laboured breathing, 4 = licking of the abdomen and 5 = stretching and contractions of the abdomen. If more than one of these behaviors was noted in one observation period, the sum of the corresponding points to the different types of behaviors was assigned; i.e., if two stretching and contractions (5 points each) and one abdominal licking (4 points) occurred during an observation period, the final score was 9 instead of 14 points. An overall score was obtained by summing the scores assigned at each time point. At the end of the 4-h observation period, referred hyperalgesia was determined by measuring the withdrawal response to a punctate mechanical stimulation of the abdomen. Forces ranging from 0.02 to 2 g (0.19-19.6 mN) were applied to the abdomen with a series of calibrated von Frey filaments (Touch-Test Sensory Evaluators, North Coast Medical Inc., CA USA) using the up-down paradigm (Chaplan et al., 1994). Filaments were applied three times for 2-3 s each one with inter-application intervals of 5 s. Testing was initiated with the 0.4 g (3.92 mN) von Frey filament, i.e., the middle of the range. In each consecutive test, if there was no response to the filament, a stronger stimulus was then selected; if there was a positive response, a weaker one was then used. The response to the filament was considered positive if immediate licking/scratching of the application site, sharp retraction of the abdomen, or jumping was observed.

The experimenter who evaluated the behavioral responses was blinded to the treatment and genotype of experimental subjects. In all cases, experiments in WT or σ₁-KO groups, solvent- or cyclophosphamide-treated groups, and saline- or drug-treatment groups were run in parallel. Each animal was used only once and received a single concentration of cyclophosphamide (or its solvent) and a single dose of one drug (or its solvent).

### 2.3 Myeloperoxidase activity determination

Changes in myeloperoxidase (MPO) activity represent a reliable index of polymorphonuclear leukocyte infiltration (Rouleau et al., 2000). Therefore, five hours after the injection of cyclophosphamide, the urinary bladder was dissected out and finely minced using spring scissors. Then it was homogenized in 0.4 ml of phosphate buffer (50 mM, pH 6) containing 0.5% hexadecyltrumethylammonium bromide (HTAB; Sigma-Aldrich). After that, they were freeze thawed three times and centrifuged (6000 g, 10 min) to collect the supernatant that was used for MPO activity assay adapted to a 96-well plate format. Briefly, 50 µl of supernatants or human neutrophil MPO standards (Sigma-Aldrich) were added to a 96-well plate. The reaction was initiated by the addition of 150 µl of phosphate buffer containing 0.167 mg/ml o-dianisidine (Sigma-Aldrich) and 0.0005% hydrogen peroxide (Sigma-Aldrich) and absorption was measured 5 min later at 450 nm (Microplate Spectrophotometer PowerWave X, Bio-tek instruments. Inc).

### 2.4 Comparison of the effect of different concentrations of cyclophosphamide in naïve WT and Sigma-1 KO mice

WT and Sigma-1-KO mice were administered with different doses of cyclophosphamide (10-300 mg/kg), and the pain-related behaviors, referred hyperalgesia to abdominal mechanical stimulation and MPO activity induced by each concentration were recorded consecutively in the same animal, following the procedure described above. This allowed the construction of dose-response curves (dose vs. pain score, mechanical threshold or MPO activity) and identification of the optimal doses of cyclophosphamide for the pharmacological studies. The results can be seen in Figure 1 and Figure 2 ((A) and (B)).

### 2.5 Comparison of drug effects on visceral pain and MPO increase induced by cyclophosphamide in WT and σ₁ KO mice

To evaluate the effect of the drugs on cyclophosphamide-induced visceral pain, the effect of several doses of various Sigma-1 receptor antagonists (Figure 4) and control drugs (Figure 5) were tested on the pain behavioral score, referred hyperalgesia and MPO activity. Thus, different doses of BD-1063 (16-64 mg/kg) (Figure 4), Example 1 (32-128 mg/kg) (Figure 4), NE-100 (16-64 mg/kg) (Figure 4), morphine (1-8 mg/kg) (Figure 5), indomethacin (2-8 mg/kg) (Figure 5) or their solvents were administered s.c. at 2 hours after the i.p. injection of cyclophosphamide and the pain behavioral score was recorded every 30 min during 2 hours. To test the effects of the drugs on the pain-related behaviors a concentration of 300 mg/kg of cyclophosphamide was administered. This concentration of cyclophosphamide was selected because it produces the maximum pain score in WT mice (see Figure 1) and therefore offers the maximum window for observing any reductions in this response. In separate experiments, we tested the effect of the same doses of the σ₁ receptor antagonists and the control drugs on the referred hyperalgesia induced by cyclophosphamide. A cyclophosphamide dose of 100 mg/kg was selected for these experiments because it reaches the maximum reduction in the mechanical threshold for referred hyperalgesia in WT and σ₁-KO mice (see Figure 6). In these experiments, the drug under study or its solvent was s.c. injected at 2 hours after the i.p. administration of cyclophosphamide, and 2 hours later (i.e., 4 hours after the cyclophosphamide injection) the response of the animal to abdominal stimulation with von Frey filaments was tested using the up-down method, as described in the general procedures (Figure 7 and Figure 8). On completion of the behavioral tests (five hours after the cyclophosphamide administration), the animals were killed and the urinary bladders were removed for determination of MPO activity Figure 9, Figure 10 and Figure 11).

To evaluate the possible modulation by Example 1 of morphine effect on referred hyperalgesia, animals received an ip injection of cyclophosphamide 100 mg/kg and 115 min later were injected with Example 1 (32 mg/kg, s.c.) or saline, 5 min later the animals were treated with morphine (1 mg/kg, s.c.) or saline and two hours after this injection the referred hyperalgesia was evaluated as described before. To test for the involvement of σ₁-receptors in Example 1-morphine interaction, PRE-084 (32 mg/kg, s.c.) was injected 5 min before Example 1 injection (Figure 12).

### 2.6 Statistical analysis

The degree of referred pain, expressed as the mechanical threshold that produces 50% of responses, was calculated using the formula of Dixon (1980): 50% mechanical threshold (g) = [(10 ^{(Xf + Kδ)}) /10.000], where X_{f} = value (in logarithmic units) of the final von Frey filament used; K = tabular value for the pattern of positive/negative responses; and δ = mean difference (in log units) between stimuli.

The mean values were compared across experimental groups with one-way or two-way analysis of variance (ANOVA) followed by the Bonferroni test, or by a Student t-test for comparisons between 2 means, using the SigmaPlot 12.0 program (Systat Software Inc., San Jose, CA, USA). P< 0.05 was considered statistically significant.

### References:

Abrahams, P. et al.; "The standardization of terminology of lower urinary tract function: report from the Standardization Sub-committee of the International Continence Society" ; Neurol. Urodyn.; 2002; 21; 167-178.
Anton, E., "Delayed toxicity of cyclophosphamide on the bladder of DBA/2 and C57BL/6 female mouse"; Int. J. Exp. Path.; 2002; 83; 47-53
Bon, K. et al.; "Characterization of cyclophosphamide cystitis, a model of visceral and referred pain, in the mouse: species and strain differences."; J Urol.; 2003; 170(3); 1008-1012
Burger "Medicinal Chemistry and Drug Discovery" 6th ed. (Donald J. Abraham ed., 2001, Wiley)
Chaplan SR, Bach FW, Pogrel JW, Chung JM, Yaksh TL; "Quantitative assessment of tactile allodynia in the rat paw". J. Neurosci. Methods; 1994; 53; 55-63.
"Design and Applications of Prodrugs" (H. Bundgaard ed., 1985, Harwood Academic Publishers
Dixon WJ; "Efficient analysis of experimental observations". Annu. Rev. Pharmacol. Toxicol.; 1980; 20; 441-62.
Dmitrieva,N. et al.; "The role of nerve growth factor in a model of visceral inflammation"; Neuroscience; 1997; 78; 449-459.
Entrena JM, Cobos EJ, Nieto FR, Cendán CM, Gris G, Del Pozo ED, Zamanillo D, Baeyens JM; "Sigma-1 receptors are essential for capsaicin-induced mechanical hypersensitivity: Studies with selective sigma-1 ligands and sigma-1 knockout mice"; Pain; 2009; 143; 252-61.
Hanno,P. et al.; "International Consultation on IC-Rome, Forging an International Consensus: progress in painful bladder syndrome/interstitial cystitis". Report and Abstracts. Int. Urogynecol. J Pelvic Floor Dysfunct.; 2005; 16(suppl. 1); S2-S34 Hellewell, S.B. and Bowen, W.D.; Brain Res. ; 1990 ; 527, 244-253
IASP, Classification of chronic pain, 2nd Edition, IASP Press (2002), 210-213
Laird JM, Souslova V, Wood JN, Cervero F; "Deficits in visceral pain and referred hyperalgesia in Nav1.8 (SNS/PN3)-null mice"; J. Neurosci.; 2002; 22; 8352-6.
Leitner, M.L. ; Eur. J. Pharmacol. ; 1994 ; 259 ; 65-69
Nakazato A, Kumagai T, Ohta K, Chaki S, Okuyama S, Tomisawa K; "Synthesis and SAR of 1-alkyl-2-phenylethylamine derivatives designed from N,Ndipropyl-4-methoxy-3-(2-phenylethoxy)phenylethylamine to discover σ1 ligands"; J. Med. Chem.; 1999; 42; 3965-70.
Olivar T, Laird JM.; "Cyclophosphamide cystitis in mice: behavioural characterisation and correlation with bladderinflammation"; Eur. J. Pain.;1999; 3;141-49.
Prasad, P.D. et al. ; J. Neurochem. ; 1998 ; 70 (2); 443-451
Quiron, R. et al. ; Trends Pharmacol. Sci. ; 1992 ; 13 : 85-86
Ronsisvalle, G. et al. ; Pure Appl. Chem. ; 2001 ; 73 ; 1499-1509
Rouleau A, Stark H, Schunack W, Schwartz JC; "Anti-inflammatory and antinociceptive properties of BP 2-94, a histamine H3-receptor agonist prodrug"; J. Pharmacol. Exp. Ther.; 2000; 295; 219-25.
Sant,G.R. et al.; "The mast cell in interstitial cyctitis : role in pathophysiology and pathogenesis"; Urology; 2007; 69(4 suppl.); 34-40.
Sonal, G. et al.; Ther. Adv. Urol.; 2011; 3(1); 19-33
Theoharides,T.C.; "Mast cell involvement in interstitial cystitis: a review of human experimental evidence"; Urology; 2001; 57 (6 suppl.); 47-55.
Van de Merwe,J.P.; "Diagnostic criteria, classification and nomenclature for painful bladder syndrome/interstitial cystitis: an ESSIC proposal"; Eur. Urol.; 2008; 53; 60-67.
Wantuch C, Piesla M, Leventhal L.; "Pharmacological validation of a model of cystitis pain in the mouse"; Neurosci. Lett.; 2007; 421; 250-2.

## Claims

1. A Sigma receptor ligand for use in the treatment and/or prevention of interstitial cystitis/bladder pain syndrome (IC/BPS) associated pain.

2. Sigma ligand according to claim 1, wherein the pain is selected from acute and/or chronic pain developed as a consequence of IC/BPS, especially neuropathic pain, neuralgia, allodynia, causalgia, hyperalgesia, hyperesthesia, hyperpathia, neuritis or neuropathy secondary to surgical procedure.

3. Sigma ligand according to any one of claims 1 to 2, which is selected from a Sigma receptor antagonist, preferably selected from a neutral antagonist, an inverse agonist and a partial antagonist.

4. Sigma ligand according to any one of claims 1 to 3, having the general formula (I): 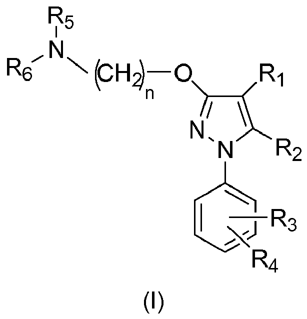 wherein
R₁ is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted non-aromatic or aromatic heterocyclyl, substituted or unsubstituted heterocyclylalkyl, -COR₈, - C(O)OR₈, -C(O)NR₈R₉, -CH=NR₈, -CN, -OR₈, -OC(O)R₈, -S(O)ₜ-R₈, -NR₈R₉, - NR₈C(O)R₉, -NO₂, -N=CR₈R₉, and halogen;
**R₂** is selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, -COR₈, - C(O)OR₈, -C(O)NR₈R₉, -CH=NR₈, -CN, -OR₈, -OC(O)R₈, -S(O)ₜ-R₈, -NR₈R₉, - NR₈C(O)R₉, -NO₂, -N=CR₈R₉, and halogen;
**R₃** and **R₄** are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, - COR₈, -C(O)OR₈, -C(O)NR₈R₉, -CH=NR₈, -CN, -OR₈, -OC(O)R₈, -S(O)ₜ-R₈, - NR₈R₉, -NR₈C(O)R₉, -NO₂, -N=CR₈R₉, and halogen, or together with the phenyl they form an optionally substituted fused ring system;
**R₅** and **R₆** are independently selected from the group consisting of hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted, aromatic or non-aromatic heterocyclyl, substituted or unsubstituted heterocyclylalkyl, - COR₈, -C(O)OR₈, -C(O)NR₈R₉, -CH=NR₈, -CN, -OR₈, -OC(O)R₈, -S(O)ₜ-R₈ , - NR₈R₉, -NR₈C(O)R₉, -NO₂, -N=CR₈R₉, and halogen, or together form, with the nitrogen atom to which they are attached, a substituted or unsubstituted, aromatic or non-aromatic heterocyclyl group;
n is selected from 1, 2, 3, 4, 5, 6, 7 and 8;
t is 0, 1 or 2;
**R₈** and **R₉** are each independently selected from hydrogen, substituted or unsubstituted alkyl, substituted or unsubstituted cycloalkyl, substituted or unsubstituted alkenyl, substituted or unsubstituted aryl, substituted or unsubstituted, aromatic or non-aromatic heterocyclyl, substituted or unsubstituted alkoxy, substituted or unsubstituted aryloxy, and halogen;
or a pharmaceutically acceptable salt, isomer, prodrug or solvate thereof.

5. Sigma ligand according to claim 4, wherein R₁ is selected from H, -COR₈ and substituted or unsubstituted alkyl.

6. Sigma ligand according to claims 4 or 5, wherein R₂ is H or substituted or unsubstituted alkyl.

7. Sigma ligand according to any one of claims 4 to 6, wherein R₃ and R₄ together form a substituted or unsubstituted fused naphthyl ring system.

8. Sigma ligand according to any one of claims 4 to 7, wherein R₅ and R₆ together form a substituted or unsubstituted morpholine-4-yl group.

9. Sigma ligand according to claim 4, which is selected from the group consisting of:
[1] 4-{2-(1-(3,4-dichlorophenyl)-5-methyl-1H pyrazol-3-yloxy)ethyl} morpholine,
[2] 2-[1-(3,4-Dichlorophenyl)-5-methyl-1H-pyrazol-3-yloxy]-N,N-diethylethanamine,
[3] 1-(3,4-Dichlorophenyl)-5-methyl-3-[2-(pyrrolidin-1-yl)ethoxy]-1H-pyrazole,
[4] 1-(3,4-Dichlorophenyl)-5-methyl-3-[3-(pyrrolidin-1-yl)propoxy]-1H-pyrazole,
[5] 1-{2-[1-(3,4-Dichlorophenyl)-5-methyl-1H-pyrazol-3-yloxy]ethyl}piperidine,
[6] 1-{2-[1-(3,4-dichlorophenyl)-5-methyl-1H-pyrazol-3-yloxy]ethyl}-1H-imidazole,
[7] 3-{1-[2-(1-(3,4-Dichlorophenyl)-5-methyl-1H-pyrazol-3-yloxy)ethyl]piperidin-4-yl}-3H-imidazo[4,5-b]pyridine,
[8]1-{2-[1-(3,4-Dichlorophenyl)-5-methyl-1H-pyrazol-3-yloxy]ethyl}-4-methylpiperazine,
[9] Ethyl 4-{2-[1-(3,4-dichlorophenyl)-5-methyl-1H-pyrazol-3-yloxy]ethyl} piperazine carboxylate,
[10] 1-(4-(2-(1-(3,4-dichlorophenyl)-5-methyl-1H-pyrazol-3-yloxy)ethyl)piperazin-1-yl)ethanone,
[11] 4-{2-[1-(4-Methoxyphenyl)-5-methyl-1H-pyrazol-3-yloxy]ethyl}morpholine,
[12] 1-(4-Methoxyphenyl)-5-methyl-3-[2-(pyrrolidin-1-yl)ethoxy]-1H-pyrazole,
[13] 1-(4-Methoxyphenyl)-5-methyl-3-[3-(pyrrolidin-1-yl)propoxy]-1H-pyrazole,
[14] 1-[2-(1-(4-Methoxyphenyl)-5-methyl-1H-pyrazol-3-yloxy)ethyl]piperidine,
[15] 1-{2-[1-(4-Methoxyphenyl)-5-methyl-1H-pyrazol-3-yloxy]ethyl}-1H-imidazole,
[16] 4-{2-[1-(3,4-Dichlorophenyl)-5-phenyl-1H-pyrazol-3-yloxy]ethyl}morpholine,
[17] 1-(3,4-Dichlorophenyl)-5-phenyl-3-[2-(pyrrolidin-1-yl)ethoxy]-1H-pyrazole,
[18] 1-(3,4-Dichlorophenyl)-5-phenyl-3-[3-(pyrrolidin-1-yl)propoxy]-1H-pyrazole,
[19] 1-{2-[1-(3,4-Dichlorophenyl)-5-phenyl-1H-pyrazol-3-yloxy]ethyl}piperidine,
[20] 1-{2-[1-(3,4-Dichlorophenyl)-5-phenyl-1H-pyrazol-3-yloxy]ethyl}-1 H-imidazole,
[21]2-{2-[1-(3,4-dichlorophenyl)-5-phenyl-1H-pyrazol-3-yloxy]ethyl}-1,2,3,4-tetrahydroisoquinoline,
[22] 4-{4-[1-(3,4-Dichlorophenyl)-5-methyl-1H-pyrazol-3-yloxy]butyl}morpholine,
[23] 1-(3,4-Dichlorophenyl)-5-methyl-3-[4-(pyrrolidin-1-yl)butoxy]-1H-pyrazole,
[24] 1-{4-[1-(3,4-Dichlorophenyl)-5-methyl-1H-pyrazol-3-yloxy]butyl}piperidine,
[25] 1-{4-[1-(3,4-Dichlorophenyl)-5-methyl-1H-pyrazol-3-yloxy]butyl}-4-methylpiperazine,
[26] 1-{4-[1-(3,4-Dichlorophenyl)-5-methyl-1H-pyrazol-3-yloxy]butyl}-1H-imidazole,
[27] 4-[1-(3,4-Dichlorophenyl)-5-methyl-1H-pyrazol-3-yloxy]-N,N-diethylbutan-1-amine,
[28]1-{4-[1-(3,4-dichlorophenyl)-5-methyl-1H-pyrazol-3-yloxy]butyl}-4-phenylpiperidine,
[29] 1-{4-[1-(3,4-dichlorophenyl)-5-methyl-1H-pyrazol-3-yloxy]butyl}-6,7-dihydro-1H-indol-4(5H)-one,
[30] 2-{4-[1-(3,4-dichlorophenyl)-5-methyl-1H-pyrazol-3-yloxy]butyl}-1,2,3,4-tetrahydroisoquinoline,
[31] 4-{2-[1-(3,4-dichlorophenyl)-5-isopropyl-1H-pyrazol-3-yloxy]ethyl}morpholine,
[32] 2-[1-(3,4-Dichlorophenyl)-5-isopropyl-1H-pyrazol-3-yloxy]-N,N-diethylethanamine,
[33] 1-(3,4-Dichlorophenyl)-5-isopropyl-3-[2-(pyrrolidin-1-yl)ethoxy]-1H-pyrazole,
[34] 1-(3,4-Dichlorophenyl)-5-isopropyl-3-[3-(pyrrolidin-1-yl)propoxy]-1H-pyrazole,
[35] 1-{2-[1-(3,4-Dichlorophenyl)-5-isopropyl-1H-pyrazol-3-yloxy]ethyl}piperidine,
[36] 2-{2-[1-(3,4-dichlorophenyl)-5-isopropyl-1H-pyrazol-3-yloxy]ethyl}-1,2,3,4-tetrahydroisoquinoline,
[37] 4-{2-[1-(3,4-dichlorophenyl)-1H-pyrazol-3-yloxy]ethyl}morpholine,
[38] 2-[1-(3,4-dichlorophenyl)-1H-pyrazol-3-yloxy] N,N-diethylethanamine,
[39] 1-(3,4-dichlorophenyl)-3-[2-(pyrrolidin-1-yl)ethoxy]-1H-pyrazole,
[40] 1-{2-[1-(3,4-dichlorophenyl)-1H-pyrazol-3-yloxy]ethyl}piperidine,
[41] 1-(3,4-dichlorophenyl)-3-[3-(pyrrolidin-1-yl)propoxy]-1H-pyrazole,
[42]1-{2-[1-(3,4-Dichlorophenyl)-5-methyl-1H-pyrazol-3-yloxy]ethyl}piperazine,
[43] 1-{2-[1-(3,4-Dichlorophenyl)-5-methyl-1H-pyrazol-3-yloxy]ethyl}pyrrolidin-3-amine,
[44]4-{2-[1-(3,4-Dichlorophenyl)-4,5-dimethyl-1H-pyrazol-3-yloxy]ethyl}morpholine,
[46]2-[1-(3,4-Dichlorophenyl)-4,5-dimethyl-1H-pyrazol-3-yloxy]-N,N-diethylethanamine,
[47] 1-(3,4-Dichlorophenyl)-4,5-dimethyl-3-[2-(pyrrolidin-1-yl)ethoxy]-1H-pyrazole,
[48] 1-(3,4-Dichlorophenyl)-4,5-dimethyl-3-[3-(pyrrolidin-1-yl)propoxy]-1H-pyrazole,
[49] 1-{2-[1-(3,4-Dichlorophenyl)-4,5-dimethyl-1H-pyrazol-3-yloxy]ethyl}piperidine,
[50] 4-{4-[1-(3,4-dichlorophenyl)-1H-pyrazol-3-yloxy]butyl}morpholine,
[51]((2S,6R)-4-{4-[1-(3,4-dichlorophenyl)-1H-pyrazol-3-yloxy]butyl}-2,6-dimethylmorpholine,
[52] 1-{4-[1-(3,4-Dichlorophenyl)-1H-pyrazol-3-yloxy]butyl}piperidine,
[53] 1-(3,4-Dichlorophenyl)-3-[4-(pyrrolidin-1-yl)butoxy]-1H-pyrazole,
[55] 4-[1-(3,4-dichlorophenyl)-1H-pyrazol-3-yloxy]-N,N-diethylbutan-1-amine,
[56] N-benzyl-4-[1-(3,4-dichlorophenyl)-1H-pyrazol-3-yloxy]-N-methylbutan-1-amine,
[57]4-[1-(3,4-dichlorophenyl)-1H-pyrazol-3-yloxy]-N-(2-methoxyethyl)-N-methylbutan-1-amine,
[58] 4-{4-[1-(3,4-dichlorophenyl)-1H-pyrazol-3-yloxy]butyl}thiomorpholine,
[59]1-[1-(3,4-Dichlorophenyl)-5-methyl-3-(2-morpholinoethoxy)-1H-pyrazol-4-yl]ethanone,
[60]1-{1-(3,4-dichlorophenyl)-5-methyl-3-[2-(pyrrolidin-1-yl)ethoxy]-1H-pyrazol-4-yl}ethanone,
[61] 1-{1-(3,4-dichlorophenyl)-5-methyl-3-[2-(piperidin-1-yl)ethoxy]-1H-pyrazol-4-yl}ethanone,
[62] 1-{1-(3,4-dichlorophenyl)-3-[2-(diethylamino)ethoxy]-5-methyl-1H-pyrazol-4-yl}ethanone,
[63] 4-{2-[5-Methyl-1-(naphthalen-2-yl)-1H-pyrazol-3-yloxy]ethyl}morpholine,
[64] N,N-Diethyl-2-[5-methyl-1-(naphthalen-2-yl)-1H-pyrazol-3-yloxy] ethanamine,
[65] 1-{2-[5-Methyl-1-(naphthalen-2-yl)-1H-pyrazol-3-yloxy]ethyl}piperidine,
[66] 5-Methyl-1-(naphthalen-2-yl)-3-[2-(pyrrolidin-1-yl)ethoxy]-1H-pyrazole, or a pharmaceutically acceptable salt, prodrug or solvate thereof.

10. Sigma ligand according to any one of claims 4 to 9, which is 4-{2-[5-methyl-1-(naphthalen-2-yl)-1H-pyrazol-3-yloxy]ethyl}morpholine, or a pharmaceutically acceptable salt, isomer, prodrug or solvate thereof.

11. Sigma ligand according to claim 10, which is 4-{2-[5-methyl-1-(naphthalen-2-yl)-1 H-pyrazol-3-yloxy]ethyl}morpholine hydrochloride.

12. Use of a Sigma ligand as defined in any one of claims 1 to 11 for the manufacture of a medicament for the treatment and/or prevention of IC/BPS associated pain.

13. A method for the treatment and/or prophylaxis of IC/BPS associated pain, which comprises administering to the patient in need of such a treatment or prophylaxis a therapeutically effective amount of a Sigma ligand as defined in any one of claims 1 to 11.

14. A combination of at least one Sigma ligand as defined in any one of claims 1 to 11 and an opioid receptor ligand for simultaneous, separate or sequential administration, for use in the treatment and/or prevention of pain associated to interstitial cystitis/painful bladder syndrome (IC/BPS).

15. The combination according to claim 14 wherein the Sigma ligand is 4-{2-[5-methyl-1-(naphthalen-2-yl)-1H-pyrazol-3-yloxy]ethyl} morpholine hydrochloride and the opioid is morphine.
